# EUROPEAN PATENT APPLICATION

(11) **EP 1 652 849 A1**
(43) Date of publication of application: **03.05.2006**
(21) Application number: 05021154.9
(22) Date of filing: 28.09.2005
(51) Int. Cl.: C07D 495/04, A61K 31/381, A61P 35/00

(54) **Novel hydrophilic analogs of 4,8-dihydrobenzodithiophene-4,8-diones as anticancer agents**

(30) Priority: 28.09.2004 US 613225 P
(71) Applicant: INDUSTRIAL TECHNOLOGY RESEARCH INSTITUTE, Chutung 310 Hsinchu (TW); Yung Shin Pharm. Ind. Co., Ltd., Tachia T'ai chung (TW)
(72) Inventor: Huang, Pi-Tsan, Hsinchu (TW); Wen, Yen-Fang, Hsinchu (TW); Shin, Wuu-Chian, Hsinchu (TW); Chen, Mei-Hwai, Hsinchu (TW); Kuo, Sheng-Chu Graduate Institute of Pharm. Chem., Taichung (TW); Lee, Kuo-Hsiung Nat. Prod. Lab. & Div. of Med., North Carolina, 27599 (US)
(74) Representative: Grünecker, Kinkeldey, Stockmair & Schwanhäusser Anwaltssozietät

(57) **Abstract**

The present invention discloses hydrophilic derivatives of 4,8-dihydrobenzodithiophene-4,8-diones of formula (D) or (E) which are active as anticancer agents, along with pharmaceutical formulations containing the same.

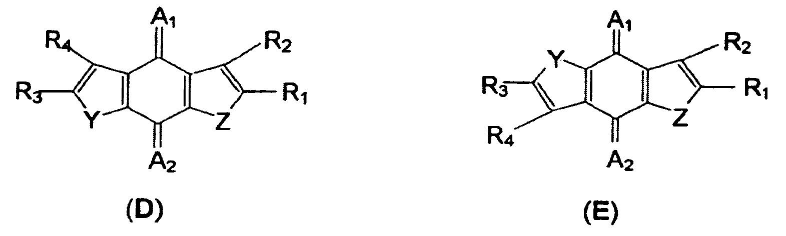

## Description

### Field of the Invention

The present invention concerns hydrophilic analogs of 4,8-dihydrobenzodithiophene-4,8-diones, which are active as anticancer agents, along with pharmaceutical formulations containing the same.

### Background of the Invention

In our prior studies, naphtho[2,3-*b*]thiophene-4,9-diones (A), 4,8-dihydrobenzo[1,2-b : 5,4-b']dithiophene-4,8-diones(B) and, 4,8-dihydrobenzo[1,2-b: 4,5-b'] dithiophene-4,8-diones(C) were identified as potential anticancer agents [US patent Nos. 6,174,913 B1; 6,337,346B1, details of which are incorporated herein by reference]. Among these benzothiophene analogs, many compounds possessed very potent cytotoxic activity against human tumor cell lines. However, most of these compounds were quite lipophilic, and therefore, not optimal for in vivo and clinical studies.

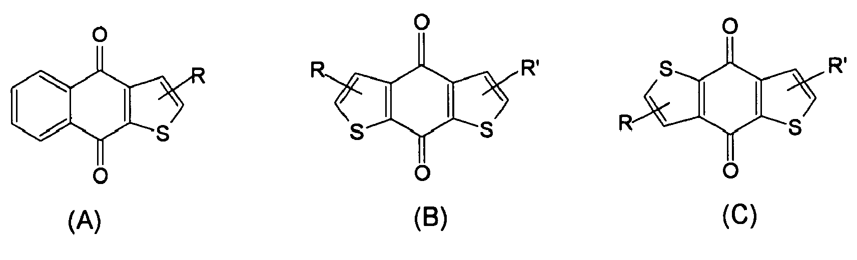

### Summary of The Invention

The present invention was undertaken to synthesize novel hydrophilic analogs of dihydrobenzodithiophenediones and to evaluate their anticancer activity.

A first aspect of the present invention is a compound selected from the group consisting of compounds of formula (D) and compounds of formula (E): 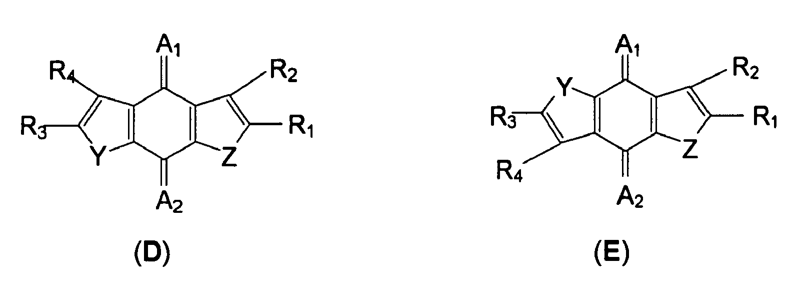 wherein Y and Z independently are O, S, -NH-, or Se, and preferably Y and Z are S;
A₁ and A₂ independently are O, S, or NR₅, wherein R₅ is H or alkyl;
R₁, R₂, R₃ and R₄ independently are H, alkyl,
-CR₅R₆-X-C(O)-(CH₂)ₙCOOH, -CR₅R₆-X-(CH₂)ₙCOOH,
-CR₅R₆-X-(CH₂)ₙNR₅R₆, -C(O)-NR₆-(CR₅R₆)ₙCOOH,
-C(O)-NR₆-(CR₅R₆)ₙNR₅R₆, -CR₅R₆-C(O)-(CH₂)ₙCOOH,
-CR₅R₆-X-C(O)-(CH₂)ₙNR₅R₆, -C(O)-(CR₅R₆)ₙCOOH, -C(O)-(CR₅R₆)ₙNR₅R₆,
-CR₅=N-(CH₂)ₙCOOH, -CR₅=N-(CH₂)ₙNR₅R₆, -CR₅=NOH, or 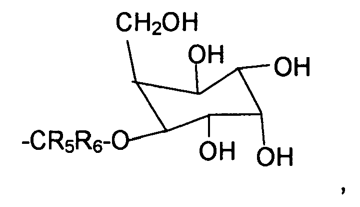 wherein X is O, S, or NH; R₅ is defined as above; R₆ is H or alkyl; and n is 1-5; subject to the proviso that at least one of R₁, R₂, R₃ and R₄ is a radical other than hydrogen and alkyl; or a pharmaceutically acceptable salt thereof.

Preferably, R₂, R₃ and R₄ are H, and R₁ is -CR₅R₆-X-C(O)-(CH₂)ₙCOOH, -CR₅R₆-X-(CH₂)ₙCOOH, -CR₅R₆-X-(CH₂)ₙNR₅R₆, -C(O)-NR₆-(CR₅R₆)ₙCOOH, or -C(O)-NR₆-(CR₅R₆)ₙNR₅R₆, wherein X, R₅, R₆ and n are defined as above.

Preferably, A₁ and A₂ are O.

Preferably, X is O.

Preferably, R₅ and R₆ independently are H or methyl.

Preferably, R₂, R₃ and R₄ are H, and R₁ is -CR₅R₆-X-C(O)-(CH₂)ₙCOOH, wherein X, R₅, R₆ and n are defined as above.

Preferably, R₂, R₃ and R₄ are H, and R₁ is -CR₅R₆-X-(CH₂)ₙCOOH, wherein X, R₅, R₆ and n are defined as above.

Preferably, R₂, R₃ and R₄ are H, and R₁ is -CR₅R₆-X-(CH₂)ₙNR₅R₆, wherein X, R₅, R₆ and n are defined as above.

Preferably, R₂, R₃ and R₄ are H, and R₁ is -C(O)-NR₆-(CR₅R₆)ₙCOOH, wherein X, R₅, R₆ and n are defined as above.

Preferably, R₂, R₃ and R₄ are H, and R₁ is -C(O)-NR₆-(CR₅R₆)ₙNR₅R₆, wherein X, R₅, R₆ and n are defined as above.

Preferably, the compound of the present invention has the formula (D).

Preferably, the compound of the present invention has the formula (E).

Preferably, the compound of the present invention has the formula (D) is mono-[1-(4,8-dioxo-4,8-dihydrobenzo[1,2-*b*:5,4-*b*'] dithiophen-2-yl)-methyl]succinate (IIa-1), mono-[1-(4,8-dioxo-4,8-dihydrobenzo[1,2-*b*:5,4-*b*'] dithiophen-2-yl)-ethyl]succinate (IIb-1), or mono-[1-(4,8-dioxo-4,8-dihydrobenzo[1,2-*b*:5,4-*b*']dithiophen-2-yl)-ethyl] pentanedioate (IIb-2); and the compound of the present invention has the formula (E) is mono-[1-(4,8-dioxo-4,8-dihydroxybenzo[1,2-*b*;4,5-*b*]dithiophen-2-yl)-ethyl] succinate (VII-1), mono-[1-(4,8-dioxo-4,8-dihydroxybenzo[1,2-*b*;4,5-*b*]dithiophen-2-yl)-ethyl] pentanedioate (VII-2), or 4,8-dioxo-4,8-dihydrobenzo[1,2-*b;*4,5-*b*]dithiophene-2-carboxylate-(2-dimethylamino-ethyl)-amide (XV-2).

A second aspect of the present invention is a composition comprising an effective anticancer amount of a compound of the formula (D) or (E) above, or a pharmaceutically acceptable salt thereof, in a pharmaceutically acceptable carrier.

A further aspect of the present invention is a method for treating a tumor, the method comprising administering to a subject in need of treatment a compound of the formula (D) or (E) above, or a pharmaceutically acceptable salt thereof, in an amount effective to treat said tumor.

A still further aspect of the present invention is the use of a compound of the formula (D) or (E) above, or a pharmaceutically acceptable salt thereof, for the preparation of a medicament for carrying out the method described above.

Preferably, said tumor is selected from the group consisting of non-small cell lung cancer, breast cancer, nasopharynx carcinoma, prostate cancer, colon cancer, hepatoma, ileocecal carcinoma, leukemia and central nervous system cancers, and more preferably said tumor is non-small cell lung cancer, breast cancer, nasopharynx carcinoma and prostate cancer.

### Detailed Description of the Invention

As part of a continuing search for potential anticancer drug candidates in the benzodithiophenedione series, novel hydrophilic analogs were synthesized and evaluated. A series of succinates (D, E) and amides (XV) derived from hydroxyalkyl-4,8-dihydrobenzo[1,2-*b*: 5,4-*b*']dithiophene-4,8-dione (I) and hydroxyalkyl-4,8-dihydrobenzo[1,2-*b*: 4,5-*b*'] dithiophene-4,8-dione **(VI)** are worthy of further exploration for their anticancer activities.

The term "alkyl" as used herein, inidividually or as a portion of another substituent term such as "alkoxy", refers to C1 to C4 alkyl, which may be linear or branched, and saturated or unsaturated. Preferably, the alkyl is saturated, and preferably the alkyl is linear.

The term "halogen" or "halo" as used herein refers to fluorine, chlorine, bromine, iodine, etc., or fluoro, chloro, bromo, iodo, etc., respectively.

Suitable methods for synthesizing the compounds of the present invention will be described in the following, and variations thereof will be apparent to those skilled in the art in given the Examples set forth below.

The key intermediates **(I,VI)** needed for the synthesis of target compounds were prepared according to our previously reported procedures [L. J. Huang, S. C. Kuo, C. Y. Perng, Y. H. Chao, T. S. Wu, A. T. Mcphail, A. Manger, H. Y. Cheng and K. H. Lee. Bioorg. Med. Chem. Lett 8, 2763-2768 (1998); Y H. Chao, S. C. Kuo, C. H. Wu, C. Y. Lee, A. Manger, I. C. Sun, S. L. Morris-Natschke and K. H. Lee. J. Med. Chem. 41, 4658-4661 (1998); Y. H. Chao, S. C. Kuo, K. Ku, I. P. Chiu, C. H. Wu, A. Manger, H. K. Wang, and K. H. Lee. Bioorg. Med. Chem. 7,1025-1031 (1999)].

As shown in Scheme 1. the mono-(4,8-dioxo-4,8-dihydrobenzo[1,2-*b* : 5,4-b'] dithiophenyl-alkyl)alkanedioates (II) were obtained by treating the key intermediate I, hydroxyalkyl-4,8-dihydrobenzo[1,2-*b* : 5,4-*b*']dithiophene-4,8-diones, with anhydride in the presence of organic or inorganic base.

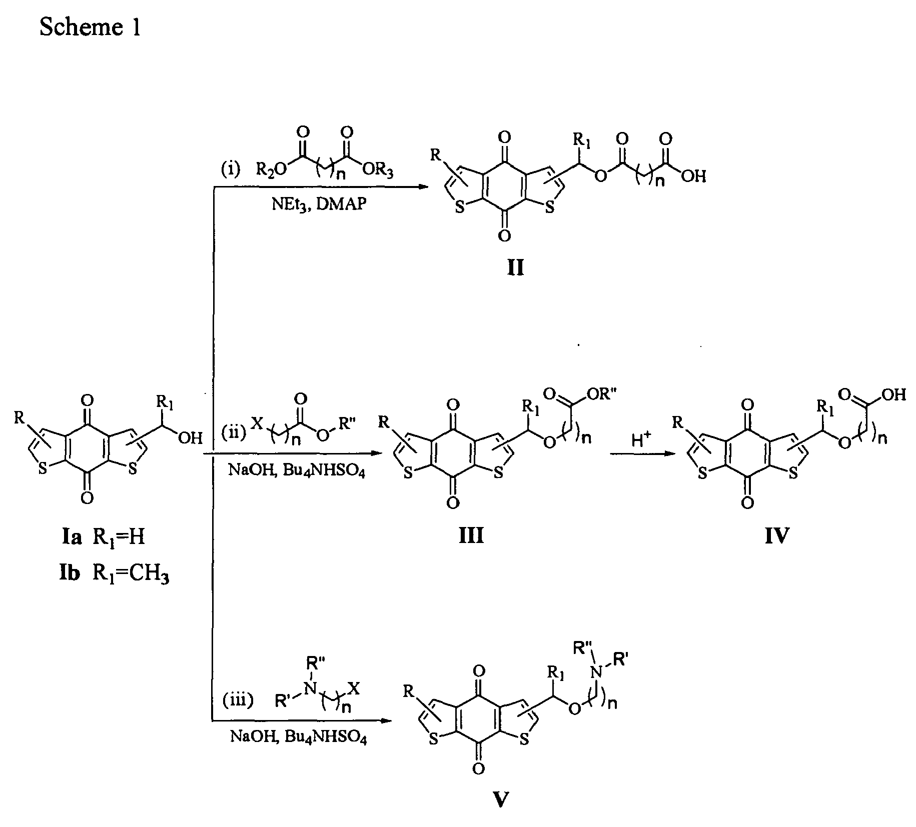

When the key intermediate I was reacted with a variety of haloalkanoates [X(CH₂)nCOOEt] in the presence of alkali, the corresponding alkoxyalkanoates (III) were obtained. Hydrolysis of compounding III with TFA or NaOH affords the corresponding acids (IV), which could be led to water soluble salts. Reaction of key intermediates I with a variety of haloalkylamines gave the corresponding alkoxyalkylamines (V), which could be treated with HCl, respectively, to afford their water soluble salts.

On the other hand, using the key intermediates VI, hydroxyalkyl-4,8-dihydrobenzo[1,2-*b* : 4,5-*b*']dithiophene-4,8-diones, as starting materials, following the similar procedures (Scheme 2) described above for the preparation of compounds II, IV, V, we successfully converted the key intermediates VI into hydrophilic target compounds VII, IX, X.

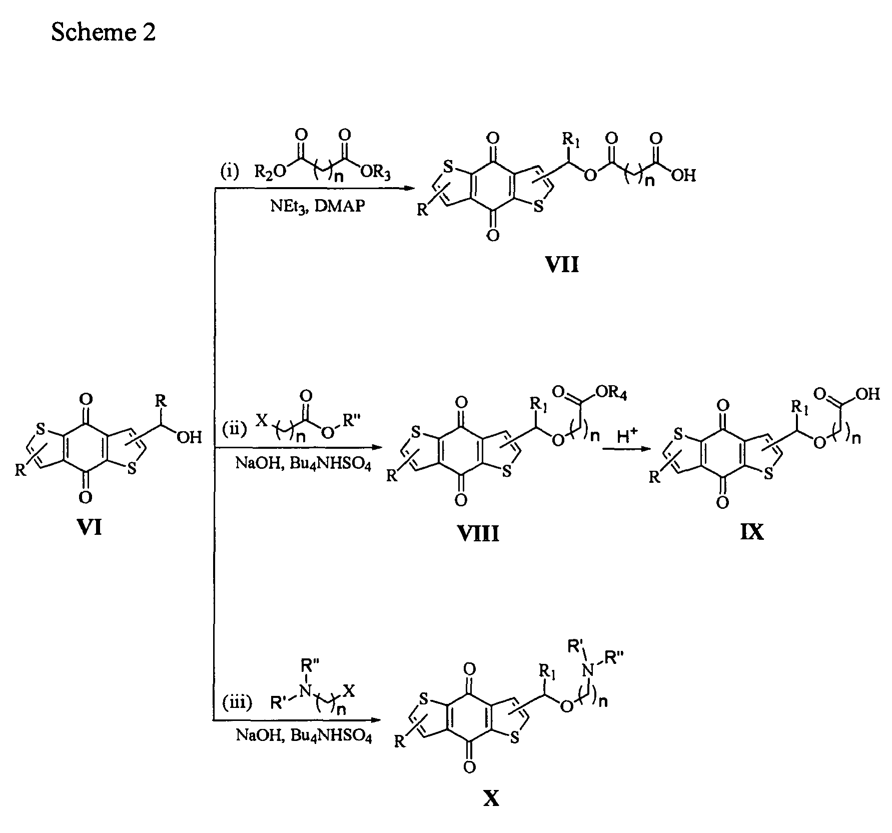

As shown in Scheme 3, the 4,8-dihydrobenzo[1,2-*b*: 5,4-b']dithio-phene-4,8-dione-2-carboxylic acid XI was treated with amino acids in the presence of coupling agent to form the amides XII, which were then converted to the corresponding acids XIII. When XI was treated with NH₃, the corresponding ammonium salts (XI-NH₄) was obtained. On the other hand, compounds XI was allowed to react with SOCl₂ to afford the corresponding acid chloride, which was treated with a variety of aminoalkylamines to give corresponding amides (XVII) which could be treated with H₃PO₄ respectively to afford their water soluble salts **(XVII-H**_{**3**}**PO**_{**4**}**).**

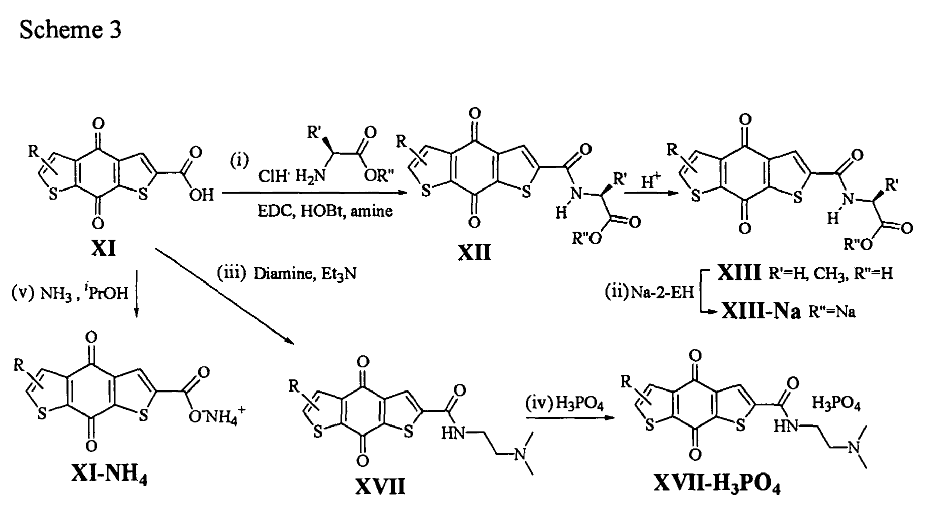

When the 4.8-dihydrobenzo[1.2-*b*: 4.5-*b*']dithiophene-4.8-dione-2-carboxylic acids (XIV) were used as starting material following the similar procedures (Scheme 4) described above for the preparation of compounds **XIII-Na ,XI-NH**_{**4**} and **XVII-H**_{**3**}**PO**_{**4**}**,** we successfully converted the starting material **XIV** into target compounds **XVI-Na** and **XIV-NH**_{**4**} and **XVII-H**_{**3**}**PO**_{**4**}**.**

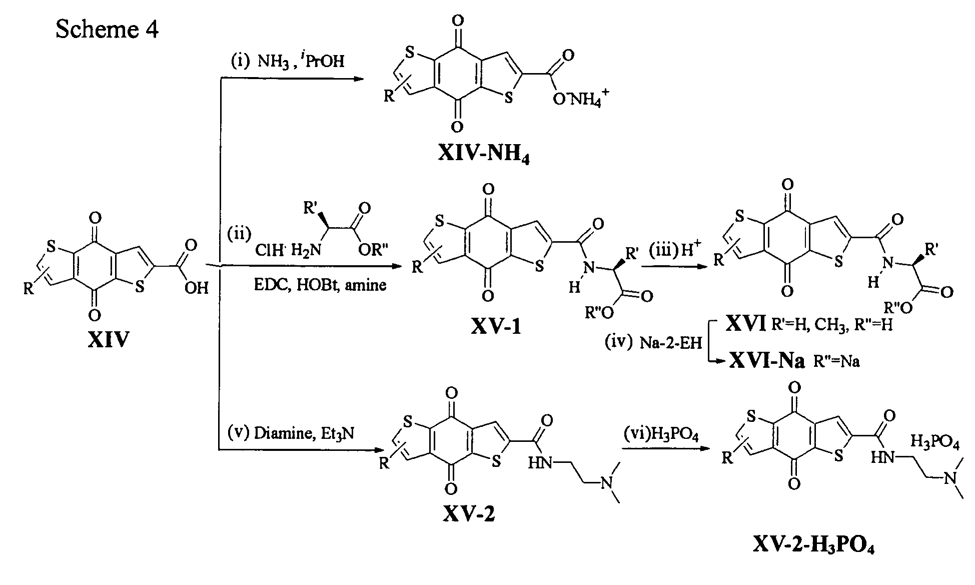

The following Examples are provided to further illustrate the present invention, and should not be construed as limiting thereof. All melting points were determined on a Büchi MP-540 apparatus and are uncorrected. NMR spectra were obtained on a Varian Unity Inova-500 spectrometer in DMSO-d6, CD₃OD or CDCl₃. The chemical shift values are expressed in δ values (parts per million). The following abbreviations are used: s =singlet, d = doublet, t = triplet, q = quartet, m = multiplet, and br = broad. Mass spectra (MS) were measured with JEOL SX102A GC-MS instrument.

### Example 1

Mono-[1-(4,8-dioxo-4,8-dihydrobenzo[1,2-*b*:5,4-*b*'] dithiophen-2-yl)-methyl]succinate **IIa-1**

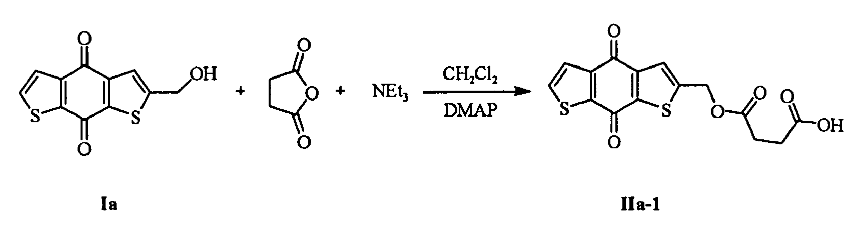

To a stirred solution of la (30mg, 0.12mmole) in CH₂Cl₂ (10ml) were added succinic anhydride (120mg, 1.2 mmole), Et₃N (25mg, 1.23mmole) and catalytic amount of DMAP (dimethyl aminopyridine). The reaction mixture was stirred at R.T. for 3 hr under N₂ and then washed with H₂O. The organic layer was extracted with aqueous NaHCO₃. To the aqueous layer was added CH₂Cl₂ and the pH was adjusted to 1 by 6N HCl. The organic layer was washed with H₂O and the volume was reduced. The solid precipitate was purified by reslurry with diethyl ether to yield yellow solid IIa-1 (13.2mg, 30%). ¹H NMR (500MHz, CDCl₃) δ 7.67 (d, *J* = 5.0Hz, 1 H), 7.60 (dd, *J* = 6.0, 5.0Hz, 1 H), 7.55 (d, *J* = 6.0Hz, 1 H), 5.32 (s, 2H), 2.70 (s, 4H).
HRMS Calcd for C₁₅H₁₀O₆S₂: 349.9919, Found: 350.9995 (MH⁺, FAB). mp= 156°C (decomp.)

### Example 2

Sodium mono-[1-(4,8-dioxo-4,8-dihydrobenzo[1,2-*b*:5,4-*b*'] dithiophen-2-yl)-methyl]succinate **IIa-1-Na**

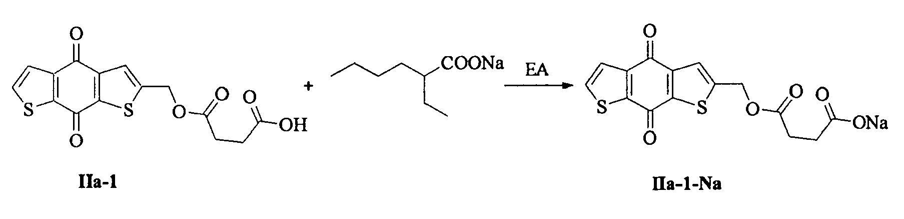

To a stirred solution of **IIa-1** (100.7mg, 0.29mmole) in EA was added sodium 2-ethylhexanoate (1.2~1.5 eq.) in EA and stirred for overnight. The precipitate was filtered and washed with EA and then dried in vacuum. Yellow solid **IIa-1-Na** (100.6mg, 93%) was obtained.
¹H NMR (500MHz, CD₃OD) δ 7.96-7.93 (m, 1 H), 7.63-7.60 (m, 2H), 5.36 (s, 2H), 2.63 (t, J = 7.0Hz, 2H), 2.48 (t, J = 7.0Hz, 2H). mp= 213°C (decomp.)

### Example 3

Mono-[1-(4,8-dioxo-4,8-dihydrobenzo[1,2-*b*:5,4-*b*'] dithiophen-2-yl)-ethyl]succinate **IIb-1**

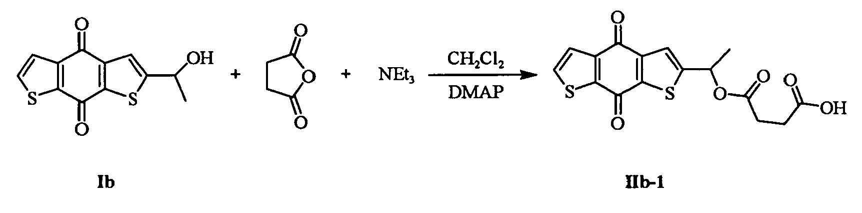

To a stirred solution of Ib (45mg, 0.18mmole) and CH₂Cl₂ (10ml) were added succinic anhydride (150mg, 1.5 mmole), Et₃N (150mg, 1.54mmole) and catalytic amount of DMAP (dimethyl aminopyridine). The reaction mixture was stirred at R.T. for 3 hr under N₂ and then washed with H₂O. The organic layer was extracted with aqueous NaHCO₃. To the aqueous layer was added CH₂Cl₂, and the pH was adjusted to 1 by 6N HCl. The separated CH₂Cl₂ layer was washed with H₂O and the volume was reduced. The solid precipitate was purified by reslurry with diethyl ether to yield yellow solid **IIb-1** (11.7mg, 17%). ¹H NMR (500MHz, CDCl₃) δ 7.66 (d, *J* = 5.0Hz, 1 H), 7.59 (d, *J* = 5.0Hz, 1 H), 7.50 (s, 1 H), 6.15 (q, J = 6.0Hz, 1 H), 2.71-2.65 (m, 4H), 1.67 (d, J = 6.0Hz, 3H).
HRMS Calcd for C₁₆H₁₂O₆S₂: 364.0075, Found: 364.0074.
mp= 132~132.5°C.

### Example 4

Sodium mono-[1-(4,8-dioxo-4,8-dihydroxybenzo-[1,2-*b*;5,4-*b*']dithiophen-2-yl)-ethyl] succinate **IIb-1-Na**

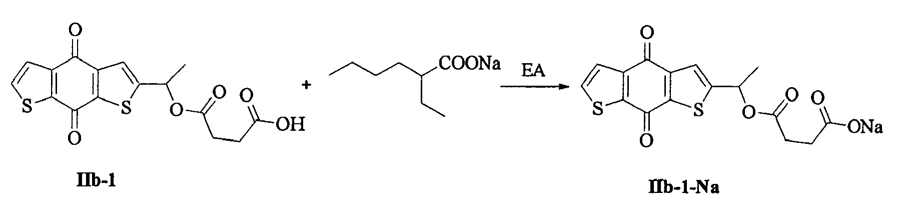

To a stirred solution of IIb-1 (100.8mg, 0.28mmole) in EA was added sodium 2-ethylhexanoate (1.2~1.5 eq.) in EA and stirred for overnight. The precipitate was filtered and washed with EA and then dried in vacuum. Yellow solid **IIb-1-Na** (96.5mg, 89%) was obtained.
¹H NMR (500MHz, CD₃OD) δ7.94 (d, J = 5.0Hz, 1 H), 7.61 (d, J = 5.0Hz, 1 H), 7.57 (s, 1 H), 6.17 (q, J = 6.5Hz, 1 H), 2.68-2.56 (m, 2H), 2.50-2.45 (m, 2H), 1.68 (d, J = 6.5Hz, 3H).
mp= 219°C (decomp.)

### Example 5

Mono-[1-(4,8-dioxo-4,8-dihydroxybenzo[1,2*-b;*4,5*-b'*]dithiophen-2-yl)-ethyl] succinate **VII-1**

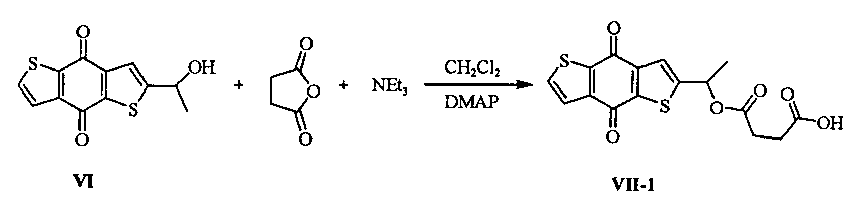

To a stirred solution of **VI** (50.0mg, 0.19mmole) and CH₂Cl₂ (3ml) were added succinic anhydride (44.8mg, 0.45mmole), Et₃N (0.10ml, 0.43mmole) and DMAP (3.0mg, 0.02mmole). The reaction mixture was stirred at R.T. for 1.5 hr under N₂. After the addition of H₂O (1ml) and 6N HCl (1ml), the reaction mixture was extracted with CH₂Cl₂ (15ml x 3). The organic layer was dried over Na₂SO₄, filtered and concentrated to yield yellow-green solid **VII-1** (62.0mg, 90%).
¹H NMR (500MHz, d₆-DMSO) δ 8.15 (d, J = 4.5Hz, 1 H), 7.63 (s, 2H), 6.14 (q, J = 6.5Hz, 1 H), 2.59-2.56 (m, 4H), 1.61 (d, J = 6.5Hz, 3H).
HRMS Calcd for C₁₆H₁₂O₆S₂: 364.0075, Found: 365.0156 (MH⁺, FAB).
mp= 163.5~164.6 °C

### Example 6

Sodium mono-[1-(4,8-dioxo-4,8-dihydroxybenzo-[1,2-*b*;4,5-*b*']dithiophen-2-yl)-ethyl] succinate **VII-1-Na**

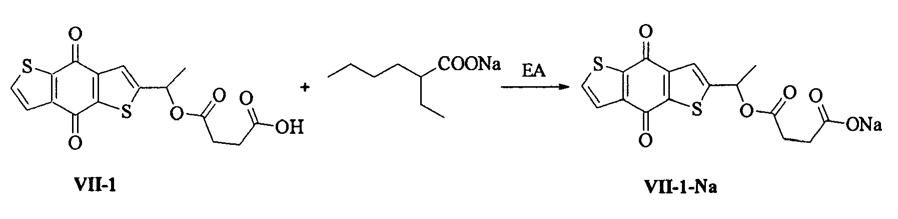

To a stirred solution of **VII-1** (200.0mg, 0.55mmole) in EA was added sodium 2-ethylhexanoate (1.2~1.5 eq.) in EA and stirred for overnight. The precipitate was filtered and washed with EA and then dried in vacuum.
Yellow-green solid **VII-1-Na** (191.0mg, 90%) was obtained.
¹H NMR (500MHz, CD₃OD) δ 7.93 (d, J = 5.0Hz, 1 H), 7.62 (d, J = 5.0Hz, 1 H), 7.57 (s, 1H). 6.17 (q, *J* = 6.5Hz, 1 H), 2.68-2.56 (m, 2H), 2.49-2.45 (m, 2H), 1.67 (d, J = 6.5Hz, 3H).
mp= 240°C (decomp.)

### Example 7

Mono-[1-(4,8-dioxo-4,8-dihydrobenzo[1,2*-b:*5,4*-b'*] dithiophen-2-yl)-methyl] pentanedioate **IIa-2**

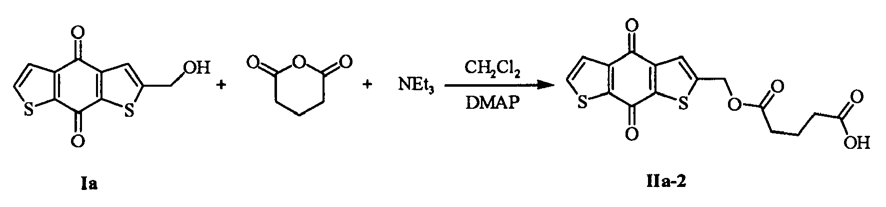

To a stirred solution of la (54.0mg, 0.22mmole) and CH₂Cl₂ (3ml) were added glutaric anhydride (49.7mg, 0.44mmole), DMAP (3.0mg, 0.02mmole) and Et₃N (0.06ml, 0.40mmole). The reaction mixture was stirred at R.T. for 3.5 hr under N₂. After the addition of H₂O (1 ml) and 2N HCl (3ml), the reaction mixture was extracted with CH₂Cl₂ (15mlx 3). The organic layer was dried over Na₂SO₄, filtered and concentrated to get crude product. It was purified by column chromatography on silica gel eluting with CH₃OH: CH₂Cl₂ (1:40) to obtain compound **IIa-2** (45.9mg, 58%).
¹H NMR (500MHz, CDCl₃) δ7.67 (d, J = 5.0Hz, 1 H), 7.61 (dd, J = 5.5, 5.0Hz, 1 H), 7.55 (d, J = 5.5Hz, 1 H), 5.29 (s, 2H), 2.48 (t, J = 7.5Hz, 2H), 2.44 (t, *J* = 7.5Hz, 2H), 2.02-1.95 (m, 2H).
HRMS Calcd for C₁₆H₁₂O₆S₂: 364.0075, Found: 364.0086.
mp= 220°C (decomp.)

### Example 8

Sodium mono-[1-(4,8-dioxo-4,8-dihydrobenzo[1,2-*b*:5,4-*b*'] dithiophen-2-yl)-methyl]pentanedioate **IIa-2-Na**

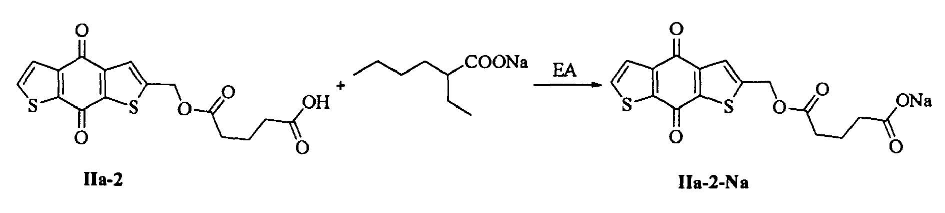

To a stirred solution of **IIa-2** (67.1 mg, 0.18mmole) in EA was added sodium 2-ethylhexanoate (1.2~1.5 eq.) in EA and stirred for overnight. The precipitate was filtered and washed with EA and then dried in vacuum. Yellowish brown solid **IIa-2-Na** (65.0mg, 94%) was obtained.
¹H NMR (500MHz, CD₃OD) δ 7.97-7.92 (m, 1H), 7.65-7.58 (m, 2H), 5.36 (s, 2H), 2.45 (t, J = 7.8Hz, 2H), 2.21 (t, J = 7.8Hz, 2H), 1.94-1.88 (m, 2H).
mp= 203°C (decomp.)

### Example 9

Mono-[1-(4,8-dioxo-4,8-dihydrobenzo[1,2-*b*:5,4-*b*'] dithiophen-2-yl)-ethyl] pentanedioate **IIb-2**

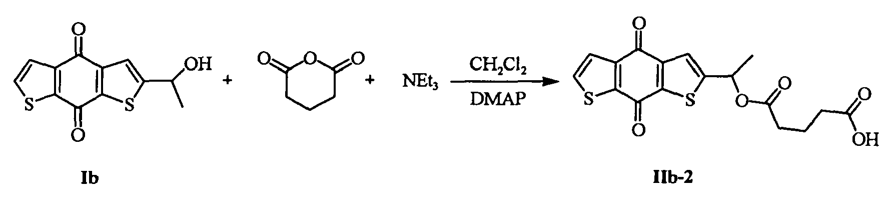

To a stirred solution of **Ib** (100.0mg, 0.4mmole) in CH₂Cl₂ (3ml) were added glutaric anhydride (136.9mg, 1.2mmole), DMAP (5.0mg, 0.04mmole) and Et₃N (121mg, 1.19mmole). The reaction mixture was stirred at R.T. for overnight under N₂. After the addition of H₂O (1ml) and 2N HCl (3ml), the reaction mixture was extracted with CH₂Cl₂ (15ml x 3). The CH₂Cl₂ layer was dried over Na₂SO₄, filtered and concentrated to get crude product. It was purified by column chromatography on silica gel eluting with CH₃OH: CH₂Cl₂ (1:40) to obtain compound **IIb-2** (128.0mg, 88%).
¹H NMR (500MHz, CDCl₃) δ7.65 (d, J = 5.0Hz, 1 H), 7.59 (d, J = 5.0Hz, 1 H), 7.49 (s, 1 H), 6.13 (q, J = 6.5Hz, 1 H), 2.47-2.40 (m, 4H), 2.00-1.93 (m, 2H), 1.66 (d, J = 6.5Hz, 3H).
HRMS Calcd for C₁₇H₁₄O₆S₂: 378.0232, Found: 378.0229.
mp= 100.5~101.0°C.

### Example 10

Sodium mono-[1-(4,8-dioxo-4,8-dihydroxybenzo[1,2-*b*;5,4-*b*]dithiophen-2-yl)-ethyl] pentanedioate **IIb-2-Na**

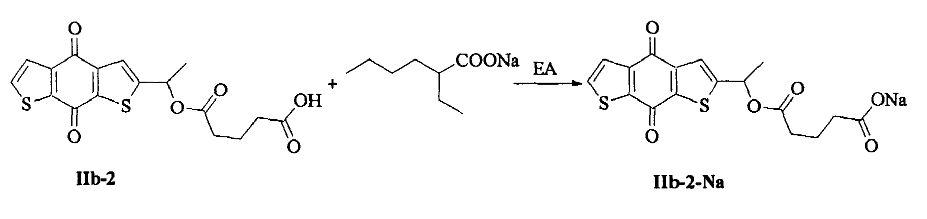

To a stirred solution of IIb-2 (83.0mg, 0.22mmole) in EA was added sodium 2-ethylhexanoate (1.2~1.5 eq.) in EA and stirred for overnight. The precipitate was filtered and washed with EA and then dried in vacuum. Yellowish brown solid **IIb-2-Na** (83.1 mg, 94%) was obtained.
¹H NMR (500MHz, CD₃OD δ 7.94 (d, *J* = 5.0Hz, 1 H), 7.62 (d, *J* = 5.0Hz, 1 H), 7.57 (d, *J* = 1.0Hz, 1 H), 6.18 (dd, *J* = 6.5, 1.0Hz, 1 H), 2.43 (td, *J* = 7.5, 2.5Hz, 2H), 2.21 (t, J = 7.5Hz, 2H), 1.94-1.88 (m, 2H), 1.67 (d, J = 6.5Hz, 3H).
mp= 228°C (decomp.)

### Example 11

Mono-[1-(4,8-dioxo-4,8-dihydroxybenzo[1,2-*b*;4,5-*b*']dithiophen-2-yl)-ethyl] pentanedioate **VII-2**

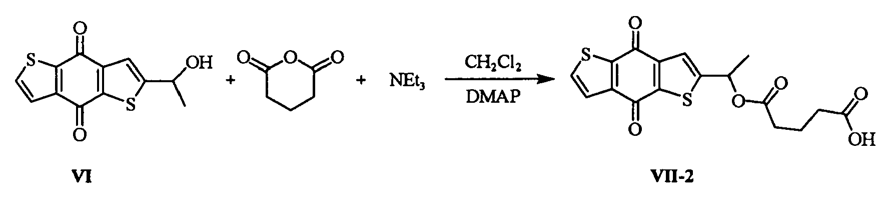

To a stirred solution of **VI** (50.0mg, 0.19mmole) in CH₂Cl₂ (3ml) were added glutaric anhydride (45.2mg, 0.4mmole), DMAP (3.0mg, 0.02mmole) and Et₃N (0.05ml, 0.4mmole). The reaction mixture was stirred at R.T. for 15 hr under N₂. After the addition of H₂O (1ml) and 2N HCl (3ml), the reaction mixture was extracted with CH₂Cl₂ (15ml×3). The CH₂Cl₂ layer was dried over Na₂SO₄, filtered and concentrated to get crude product. It was purified by column chromatography on silica gel eluting with CH₃OH: CH₂Cl₂ (1:50) to obtain compound **VII-2** (32.7mg, 46%).
¹H NMR (500MHz, CDCl₃) δ 7.65 (d, J = 5.0Hz, 1 H), 7.61 (d, J = 5.0Hz, 1 H), 7.51 (s, 1 H), 6.13 (q, J = 6.5Hz, 1 H), 2.47-2.41 (m, 4H), 2.00-1.94 (m, 2H), 1.66 (d, J = 6.5Hz, 3H).
HRMS Calcd for C₁₇H₁₄O₆S₂: 378.0232, Found: 379.0313 (MH⁺, FAB).
mp= 141.6~142.4°C

### Example 12

Sodium mono-[1-(4,8-dioxo-4,8-dihydroxybenzo[1,2-*b*;4,5-*b*']dithiophen-2-yl)-ethyl] pentanedioate **VII-2-Na**

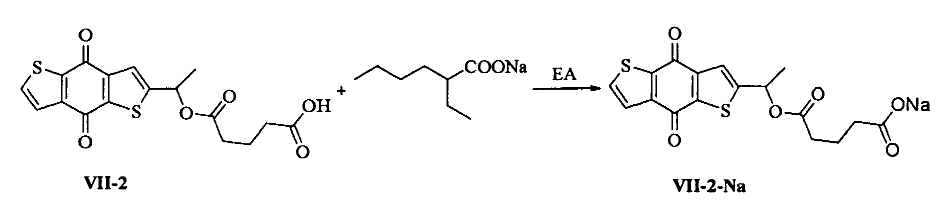

To a stirred solution of **VII-2** (109.4mg, 0.29mmole) in EA was added sodium 2-ethylhexanoate (1.2~1.5 eq.) in EA and stirred for overnight. The precipitate was filtered and washed with EA and then dried in vacuum.
Yellow-green solid **VII-2-Na** (79.0mg, 68%) was obtained.
¹H NMR (500MHz, CD₃OD) δ 7.93 (d, *J* = 5.0Hz, 1 H), 7.62 (d, *J* = 5.0Hz, 1 H), 7.57 (s, 1 H), 6.17 (q, *J* = 6.5Hz, 1 H), 2.44 (td, *J* = 7.5, 2.5Hz, 2H), 2.22 (t, *J* = 7.5Hz, 2H), 1.95-1.88 (m, 2H), 1.68 (d, J = 6.5Hz, 3H).
mp= 230°C (decomp.)

### Example 13

*t*-Butyl 4,8-dioxo-4,8-dihydroxybenzo[1,2-*b*:5,4-*b*']-dithiophen-2-yl-methoxy acetate **IIIa-1**

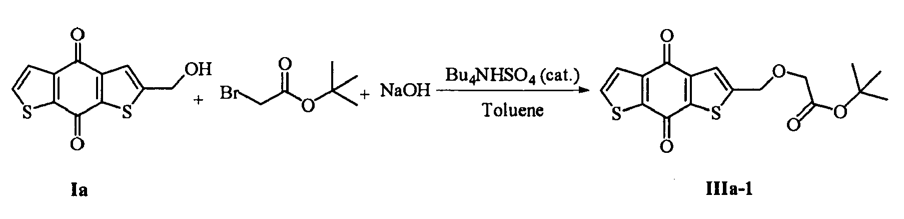

To a stirred solution of compound la (9.4mg, 0.04mmole) in toluene (2ml) were added tetrabutylammonium hydrogen sulfate (4.0mg, 0.01 mmole), 50% NaOH_{(aq.)} (1ml) and *t*-butyl bromoacetate (0.02ml, 0.1mmole). The mixture was stirred at R.T. for 2 hr. After the addition of H₂O, the reaction mixture was extracted with CH₂Cl₂ (15mlx 3). The CH₂Cl₂ layer was dried over Na₂SO₄, filtered and concentrated to get crude product. It was purified by plate liquid chromatography eluting with Ethyl acetate : Hexane (1:8) to obtain yellow solid **IIIa-1** (2.7mg, 20 %).
¹H NMR (500MHz, CDCl₃) δ 7.65 (d, *J* = 5.0Hz, 1 H), 7.60 (dd, *J* = 5.5, 5.0Hz, 1 H), 7.49 (d, J = 5.5Hz, 1 H), 4.83 (s, 2H), 4.05 (s, 2H), 1.48 (s, 9H).
HRMS Calcd for C₁₇H₁₆O₅S₂: 364.0439, Found: 364.0425
mp= 97.2~97.8°C

### Example 14

Ethyl 4,8-dioxo-4,8-dihydroxybenzo[1,2-*b*:5,4-*b*]-dithiophen-2-yl-methoxy acetate **IIIa**

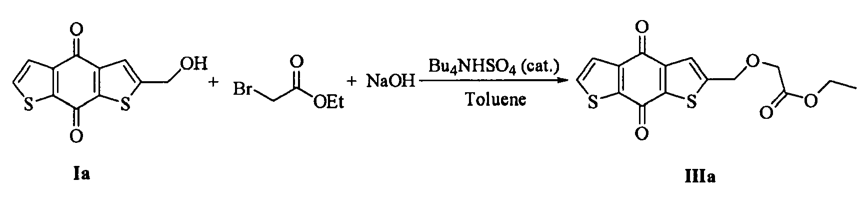

To a stirred solution of compound **la** (40mg, 0.17mmole) and toluene (10ml) were added tetrabutylammonium hydrogen sulfate (20mg, 0.06mmole), 50% NaOH_{(aq.)} (1 ml) and ethyl bromoacetate (0.1 ml, 0.9mmole). The mixture was stirred at R.T. for 2 hr. After the addition of H₂O, the reaction mixture was extracted with CH₂Cl₂ (25mlx 3). The CH₂Cl₂ layer was dried over Na₂SO₄, filtered and concentrated to get crude product. It was purified by plate liquid chromatography eluting with CH₂Cl₂ to obtain yellow solid **IIIa** (18mg, 33 %).
¹H NMR (500MHz, CDCl₃) δ7.65 (d, J = 5.0Hz, 1 H), 7.62-7.59 (m, 1H), 7.51-7.49 (m, 1 H), 4.85 (s, 2H), 4.24 (q, J = 7.0Hz, 2H), 4.16 (s, 2H), 1.29 (t, *J* = 7.0Hz, 3H).
HRMS Calcd for C₁₅H₁₂O₅S₂: 336.0126, Found: 336.0125.
mp= 113.3 ~ 113.5°C

### Example 15

4,8-dioxo-4,8-dihydroxybenzo[1,2-*b*:5,4-*b*']-dithiophen-2-yl-methoxy acetic acid **IVa**

### ◆ Method A

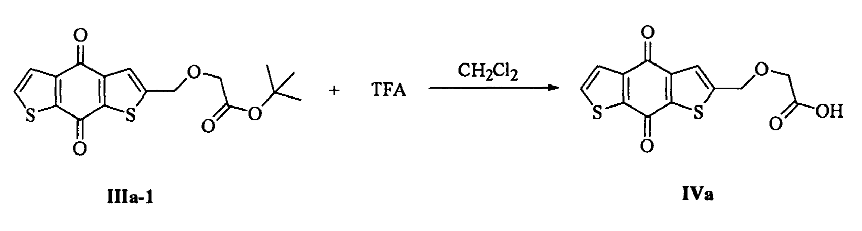

To a stirred solution of compound **IIIa-1** (2.7mg, 0.007mmole) and CH₂Cl₂ (1ml) was added trifluoroacetic acid (0.1ml) and stirred at R.T. for 1.5 hr under N₂. After concentration, yellow solid **IVa** was obtained.

### ◆ Method B

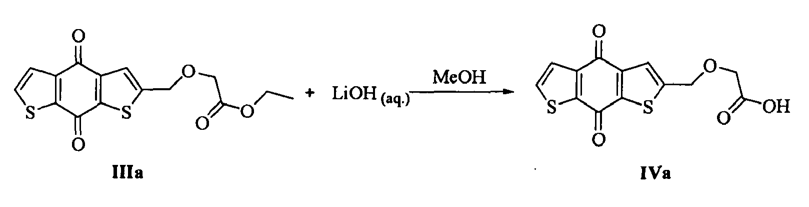

In a 25mL flask were placed compound **IIIa** (15mg, 0.05mmole) and MeOH (10ml). LiOH (10mg) in H₂O (8ml) was then added and reaction mixture stirred at R. T. until hydrolysis completed. HOAc (~0.4ml) was added drop-wise to neutralize. MeOH was removed under vacuum and the residue was treated with aq. Na₂CO₃ and then extracted with CH₂Cl₂. The aqueous layer was acidified with dilute HCl solution until pH~4 and the extracted with CH₂Cl₂. The CH₂Cl₂ layer was dried with Na₂SO₄, filtered and concentrated to obtain yellow solid **IVa** (12.5mg, 91 %).
¹H NMR (500MHz, d₆-DMSO) δ 8.15 (d, J = 5.0Hz, 1 H), 7.62 (dd, J = 5.0, 4.5Hz, 1 H), 7.57 (d, J = 4.5Hz, 1 H), 6.47 (s, 1 H), 4.85 (s, 2H), 4.16 (s, 2H). HRMS Calcd for C₁₃H₈O₅S₂: 307.9813, Found: 307.9847.
mp= 218.0 ~ 218.2°C

### Example 16

Sodium 4,8-dioxo-4,8-dihydroxybenzo[1,2-*b*:5,4-*b*']-dithiophen-2-yl-methoxy acetate **IVa-Na**

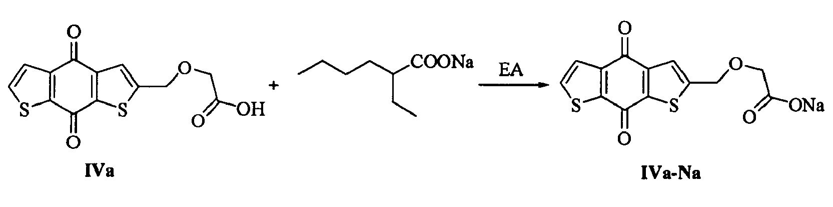

To a stirred solution of **IVa** (47.8mg, 0.16mmole) in EA was added sodium 2-ethylhexanoate (1.2~1.5 eq.) in EA and stirred for overnight. The precipitate was filtered and washed with EA and then dried in vacuum.
Yellow-green solid **IVa -Na** (35.4mg, 70%) was obtained.
¹H NMR (500MHz, CD₃OD) δ7.93 (br, 1H), 7.62 (br, 1 H), 7.54 (s, 1 H), 4.88 (s, 2H), 3.97 (s, 2H).
mp= 232.7°C (decomp.)

### Example 17

*t*-Butyl 1-(4,8-dioxo-4,8-dihydroxybenzo[1,2-*b*:5,4-*b*']-dithiophen-2-yl-ethoxy)-acetate **IIIb-1**

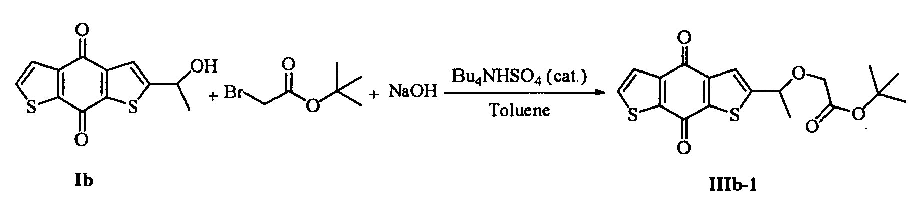

To a stirred solution of compound Ib (9.4mg, 0.04mmole) in toluene (2ml) were added tetrabutylammonium hydrogen sulfate (4.0mg, 0.01mmole), 50% NaOH_{(aq.)} (1ml) and *t*-butyl bromoacetate (0.02ml, 0.1mmole). The mixture was stirred at R.T. for 2 hr. After the addition of H₂O, the reaction mixture was extracted with CH₂Cl₂ (15ml x 3), and the organic layer was dried over Na₂SO₄, filtered and concentrated to get crude product. It was purified by plate liquid chromatography eluting with ethyl acetate: Hexane (1:15) to obtain yellow solid **IIIb-1** (6.9mg, 51 %).
¹H NMR (500MHz, CDCl₃) δ7.60 (d, J = 5.0Hz, 1 H), 7.595 (d, J = 5.0Hz, 1 H), 7.45 (s, 1H), 4.88 (q, J = 6.5Hz, 1 H), 4.02, 3.91 (ABq, J = 16.5Hz, 2H), 1.62 (d, J = 6.5Hz, 3H), 1.46 (s, 9H).
HRMS Calcd for C₁₈H₁₈O₅S₂: 378.0596, Found: 378.0587
mp= 108.0~108.7°C

### Example 18

Ethyl 1-(4,8-dioxo-4,8-dihydroxybenzo[1,2-*b*:5,4-*b*']-dithiophen-2-yl-ethoxy)-acetate **lllb**

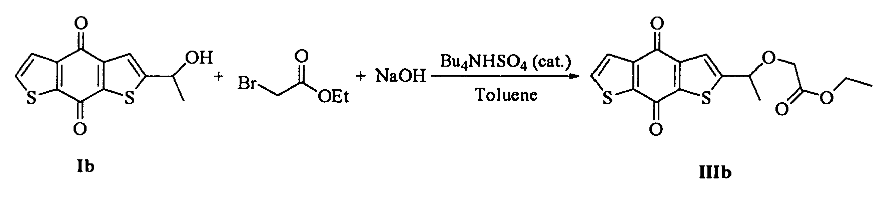

To a stirred solution of compound Ib (100mg, 0.4mmole) in toluene (20ml) were added tetrabutylammonium hydrogen sulfate (40mg, 0.11 mmole), 50% NaOH_{(aq.)} (1ml) and ethyl bromoacetate (0.2ml, 1.8mmole). The mixture was stirred at R.T. for 2 hr. After the addition of H₂O, the reaction mixture was extracted with CH₂Cl₂ (20mlx 3). The CH₂Cl₂ layer was dried over Na₂SO₄, filtered and concentrated to get crude product. It was purified by plate liquid chromatography eluting with CH₂Cl₂ to obtain yellow solid IIIb (65mg, 48 %).
¹H NMR (500MHz, CDCl₃) δ7.65 (d, J = 5.0Hz, 1 H), 7.60 (d, J = 5.0Hz, 1 H), 7.46 (s, 1 H), 4.89 (q, J = 6.5Hz, 1 H), 4.21 (q, J = 7.0Hz, 2H), 4.12, 4.02 (ABq, J = 16.5Hz, 2H), 1.63 (d, J = 6.5Hz, 3H), 1.27 (t, J = 7.OHz, 3H).
HRMS Calcd for C₁₆H₁₄O₅S₂: 350.0283, Found: 350.0271.
mp= 122.7~122.9°C

**Example 19** 1-(4,8-Dioxo-4,8-dihydroxybenzo[1,2-b:5,4-b]-dithiophen-2-yl-ethoxy)-acetic acid **IVb**

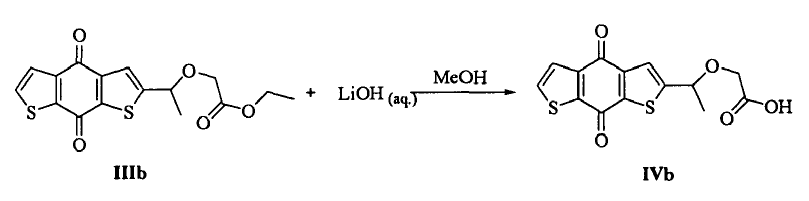

In a 100mL flask were placed compound lllb (65mg, 0.19mmole) and MeOH (15ml). LiOH (25.0mg) in H₂O (12ml) was then added and reaction mixture stirred at R.T. until hydrolysis completed. HOAc (~1.4ml) was added drop-wise to neutralize. MeOH was removed under vacuum and the residue was treated with aq. Na₂CO₃ and then extracted with CH₂Cl₂. The aqueous layer was acidified with dilute HCl solution until pH~4 and the extracted with CH₂Cl₂. The CH₂Cl₂ layer was dried with Na₂SO₄, filtered and concentrated to obtain yellow solid **IVb** (54mg, 90 %).
¹H NMR (500MHz, CD₃ OD) δ7.95 (d, J = 5.0Hz, 1 H), 7.62 (d, J = 5.0Hz, 1 H), 7.53 (s, 1 H), 4.98 (q, J = 6.5Hz, 1 H), 4.15, 4.09 (ABq, J = 16.5Hz, 2H), 1.61 (d, J = 6.5Hz, 3H).
HRMS Calcd for C₁₄H₁₀O₅S₂: 321.9970, Found: 321.9994.
mp= 172.6~173.0°C

### Example 20

Sodium 1-(4,8-dioxo-4,8-dihydroxybenzo[1,2-*b*:5,4-*b*']-dithiophen-2-yl-ethoxy)-acetate **IVb-Na**

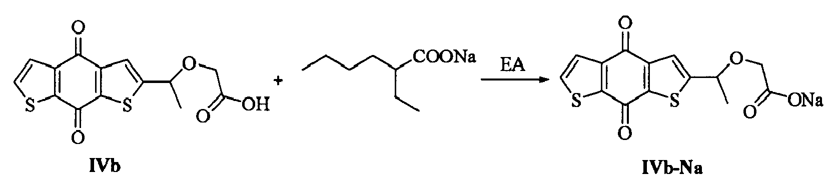

To a stirred solution of **IVb** (53.0mg, 0.16mmole) in EA was added sodium 2-ethylhexanoate (1.2~1.5 eq.) in EA and stirred for overnight. The precipitate was filtered and washed with EA and then dried in vacuum.
Yellow-green solid **IVb -Na** (33.1 mg, 58%) was obtained.
¹H NMR (500MHz, D₂O) δ 7.87 (d, *J* = 5.0Hz, 1 H), 7.32 (d, *J* = 5.0Hz, 1 H), 7.27 (s, 1 H), 4.98 (q, *J* = 6.5Hz, 1 H), 4.02, 3.94 (ABq, *J* = 16.0Hz, 2H), 1.64 (d, J = 6.5Hz, 3H).
mp= 243.8~244.2°C.

### Example 21

*t*-Butyl 1-(4,8-dioxo-4,8-dihydroxybenzo[1,2-*b*:4,5-*b*']-dithiophen-2-yl-ethoxy)-acetate **VIII-1**

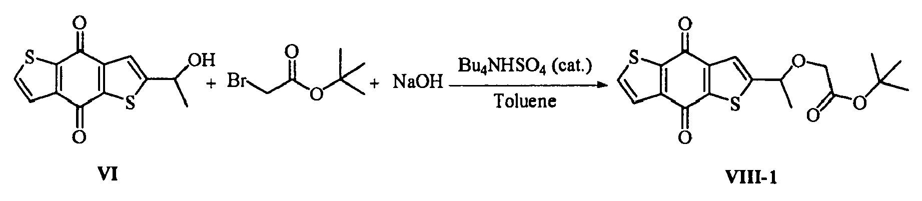

To a stirred solution of compound VI (9.0mg, 0.03mmole) in toluene (2ml) were added tetrabutylammonium hydrogen sulfate (4.0mg, 0.01 mmole), 50% NaOH_{(aq.)} (1 ml) and *t*-butyl bromoacetate (0.02ml, 0.1mmole). The mixture was stirred at R.T. for 2 hr. After the addition of H₂O, the reaction mixture was extracted with CH₂Cl₂ (15ml× 3), and the organic layer was dried over Na₂SO₄, filtered and concentrated to get crude product. It was purified by plate liquid chromatography eluting with ethyl acetate: Hexane (1:15) to obtain yellow oil VIII-1 (5.0mg, 39 %).
¹H NMR (500MHz, CDCl₃) δ7.65 (d, J = 5.0Hz, 1 H), 7.61 (d, J = 5.0Hz, 1 H), 7.46 (s, 1 H), 4.87 (q, J = 6.5Hz, 1 H), 4.01, 3.90 (ABq, J = 16.5Hz, 2H), 1.62 (d, J = 6.5Hz, 3H), 1.46 (s, 9H).
HRMS Calcd for C₁₈H₁₈O₅S₂: 378.0596, Found: 378.0573

### Example 22

Ethyl 1-(4,8-dioxo-4,8-dihydroxybenzo[1,2-*b*:4,5-*b*']-dithiophen-2-yl-ethoxy)-acetate **VIII**

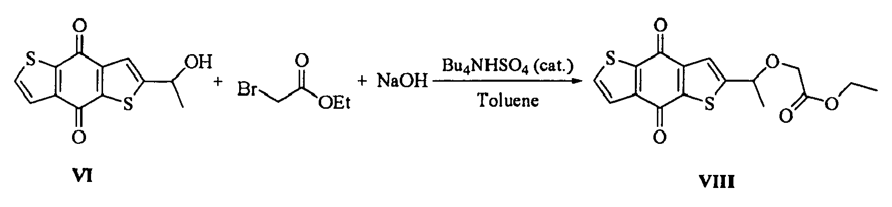

To a stirred solution of compound VI (9.7mg, 0.04mmole) and toluene (2ml) were added tetrabutylammonium hydrogen sulfate (4.0mg, 0.01 mmole), 50% NaOH_{(aq.)} (1ml) and ethyl bromoacetate (0.1mmole). The mixture was stirred at R.T. for 2 hr. After the addition of H₂O, the reaction mixture was extracted with CH₂Cl₂(15ml× 3). The CH₂Cl₂ layer was dried over Na₂SO₄, filtered and concentrated to get crude product. It was purified by plate liquid chromatography eluting with CH₂Cl₂ to obtain yellow solid VIII (2.5mg, 19 %).
¹H NMR (500MHz, CDCl₃)δ7.65 (d, J = 5.0Hz, 1H), 7.61 (d, J = 5.0Hz, 1 H), 7.47 (s, 1 H), 4.89 (q, J = 6.5Hz, 1 H), 4.21 (q, J = 7.0Hz, 2H), 4.12, 4.02 (ABq, J = 16.5Hz, 2H), 1.63 (d, J = 6.5Hz, 3H), 1.27 (t, J = 7.0Hz, 3H).
HRMS Calcd for C₁₆H₁₄O₅S₂: 350.0283, Found: 350.0284.
mp= 106.2~107.1°C

### Example 23

1-(4,8-Dioxo-4,8-dihydroxybenzo[1,2-*b*:4,5-*b*']-dithiophen-2-yl-ethoxy)-acetic acid **IX**

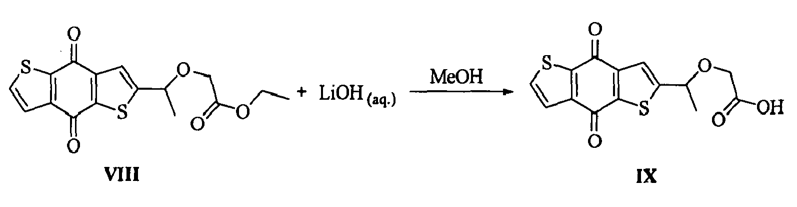

In a 25mL flask were placed compound VIII (192.7mg, 0.55mmole) and MeOH (30ml). LiOH (0.36g) in H₂O (30ml) was then added and reaction mixture stirred at R. T. until hydrolysis completed. HOAc (~1.1 ml) was added drop-wise to neutralize. MeOH was removed under vacuum and the residue was treated with aq. Na₂CO₃ and then extracted with EA. The aqueous layer was acidified with dilute HCl solution until pH~4 and the extracted with EA. The EA layer was dried with Na₂SO₄, filtered and concentrated to obtain yellow solid IX (160.0mg, 90 %).
¹H NMR (500MHz, CD₃ OD) δ7.90 (d, J = 5.0Hz, 1 H), 7.60 (d, J = 5.0Hz, 1H), 7.51 (s, 1 H), 4.96 (q, J = 6.5Hz, 1H), 4.15, 4.09 (ABq, J = 16.5Hz, 2H), 1.61 (d, J = 6.5Hz, 3H).
HRMS Calcd for C₁₄H₁₀O₅S₂: 321.9970, Found: 323.0054 (MH⁺, FAB).
mp= 172.1~172.8 °C

### Example 24

Sodium 1-(4,8-dioxo-4,8-dihydroxybenzo[1,2-*b*:4,5-*b*']-dithiophen-2-yl-ethoxy)-acetate **IX-Na**

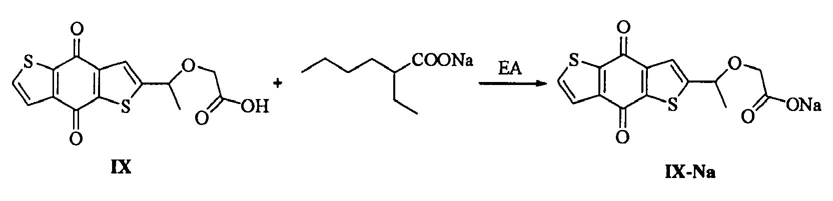

To a stirred solution of IX (85.0mg, 0.28mmole) in EA was added sodium 2-ethylhexanoate (1.2~1.5 eq.) in EA and stirred for overnight. The precipitate was filtered and washed with EA and then dried in vacuum. Yellow solid
**IX-Na** (83.0mg, 92%) was obtained.
¹H NMR (500MHz, CD₃ OD) δ7.92 (d, J = 5.0Hz, 1 H), 7.62 (d, J = 5.0Hz, 1 H), 7.52 (s, 1 H), 4.96 (q, J = 6.5Hz, 1 H), 3.92, 3.83 (ABq, J = 15.5Hz, 2H), 1.60
(d, J = 6.5Hz, 3H).
mp= 223°C (decomp.)

### Example 25

2-[1-(2-Pyrrol-1-yl-propoxy)-ethylbenzo[1,2-*b*:4,5-*b*']dithiophen-4,8-dione X

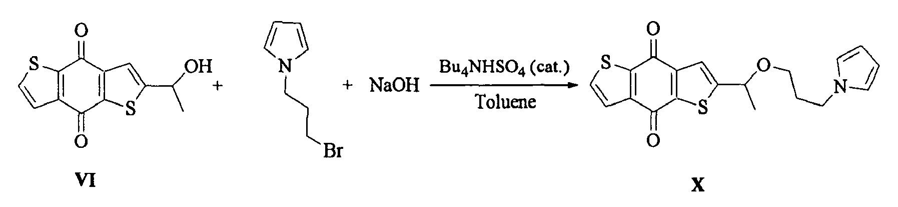

To a stirred solution of compound VI (10.5mg, 0.04mmole) in toluene (2ml) were added tetrabutylammonium hydrogen sulfate (4.0mg, 0.01 mmole), 50% NaOH_{(aq.)} (2ml) and 1-(3-bromopropyl)pyrrole (0.1mmole). The mixture was stirred at R.T. for 3 hr. After the addition of H₂O, the reaction mixture was extracted with CH₂Cl₂ (15ml× 3). The CH₂Cl₂ layer was dried over Na₂SO₄, filtered and concentrated to get crude product. It was purified by plate liquid chromatography eluting with CH₂Cl₂ to obtain yellow oil X (5.0mg, 34 %).
¹H NMR (500MHz, CDCl₃) δ7.65 (d, J = 5.0Hz, 1 H), 7.61 (d, J = 5.0Hz, 1 H), 7.41 (s, 1 H), 6.61 (s, 2H), 6.10 (s, 2H), 4.64 (q, J = 6.5Hz, 1 H), 4.06-3.94 (m, 2H), 3.38 (t, J = 5.5Hz, 2H), 2.04-1.99 (m, 2H), 1.56 (d, J = 6.5Hz, 3H). HRMS Calcd for C₁₉H₁₇NO₃S₂: 371.0650, Found: 371.0663

### Example 26

4,8-dioxo-4,8-dihydrobenzo [1,2-*b*;5,4-*b*]dithiophene-2-carboxylate-(2-dimethylamino-ethyl)-amide **XVII**

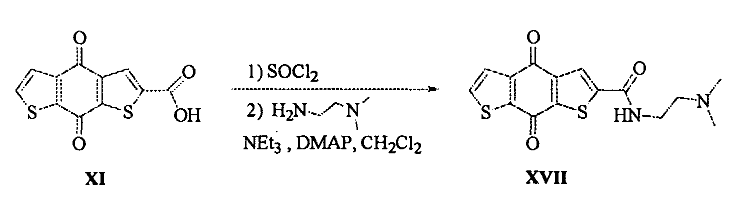

Compound **XI** (60mg, 0.23mmole) in SOCl₂ (5ml) was heated to reflux for 2 hrs. The excess SOCl₂ was removed to yield crude acid chloride. The acid chloride obtained was used directly without purification. It was diluted with CH₂Cl₂ (4ml) and added to the N,N-dimethylethylenediamine (1.5ml), Et₃N (2ml) and CH₂Cl₂ (4ml) mixture and stirred at R.T. for 1.5~2 hrs. Water was added and the reaction mixture extracted with CH₂Cl₂. The CH₂Cl₂ layer was concentrated and crude product was purified through column using MeOH: CH₂Cl₂ =1:8 as the eluent. Yellow solid **XVII** (50.0mg, 67 %) was obtained.
¹H NMR (500MHz, CD₃OD) δ 8.14 (d, J = 2.0Hz, 1 H), 7.97~8.00 (m, 1 H), 7.65 (d, J = 5.0Hz, 1 H), 3.54 (t, J = 6.5Hz, 2H), 2.59 (t, J = 6.5Hz, 2H), 2.33 (s, 6H).
HRMS Calcd for C₁₅H₁₄N₂O₃S₂: 334.0446, Found: 334.0435
mp= 197.5~198.0°C

### Example 27

4,8-dioxo-4,8-dihydrobenzo [1,2-*b*;5,4-*b*']dithiophene-2-carboxylate-(2-dimethyl-aminoethyl)-amide phosphoric acid salt **XVII-H**_{**3**}**PO**_{**4**}

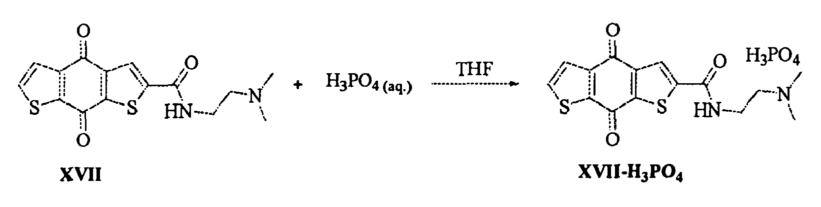

To a stirred solution of **XVII** (31 mg, 0.093mmole) in THF was added 85% H₃PO₄ (1.2~1.5 eq.) and stirred for overnight. The precipitate was filtered and washed with THF and then dried in vacuum. Yellow solid **XVII-H**_{**3**}**PO**_{**4**} (39mg, 97%) was obtained.
¹H NMR (500MHz, D₂O) δ 8.16 (s, 1 H), 8.02 (d, J = 5.0Hz, 1 H), 7.66 (d, J = 5.0Hz, 1 H), 3.78 (t, J = 6.0Hz, 2H), 3.37 (t, J = 6.0Hz, 2H), 2.97 (s, 6H).
mp= 222.5°C (decomp.)

### Example 28

(a) Ammonium 4,8-dioxo-4,8-dihydrobenzo-[1,2-*b*:5,4-*b*']dithiophene-2-carboxylate **XI-NH**_{**4**} 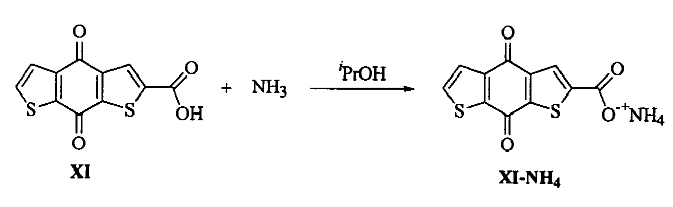 Aqueous ammonium hydroxide (29.5%) was added to the solution of compound **XI** (230mg, 0.87mmole) in IPA (40mL) until basic. After stirring for a while, all the volatile solvent was removed to yield green-yellow solid
   **XI-NH**_{**4**} (200mg, 82%)
   ¹H NMR (500MHz, CD₃OD) δ 7.94 (d, *J* = 5.0Hz, 1 H), 7.91 (s, 1 H), 7.62 (d, *J*
   = 5.0Hz, 1 H).
   mp= 257~259°C.
(b) Sodium 4,8-dioxo-4,8-dihydrobenzo-[1,2-*b*:5,4-*b*']dithiophene-2-carboxylate **XI-Na**

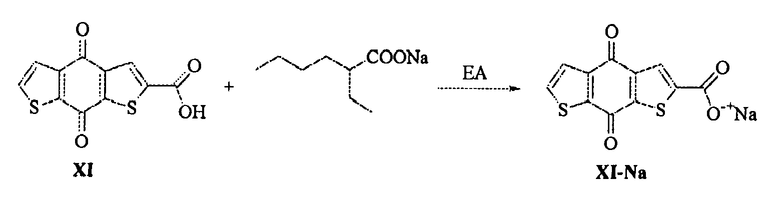

To a stirred solution of XI (206.7mg, 0.83mmole) in EA was added sodium 2-ethylhexanoate (1.2~1.5 eq.) in EA and stirred for overnight. The precipitate was filtered and washed with EA and then dried in vacuum. Yellowish brown solid **XI-Na** (202.5mg, 90%) was obtained.
¹H NMR (500MHz, CD₃OD) δ 7.93 (dd, J = 5.0, 2.0Hz, 1 H), 7.90 (d, J = 2.0Hz, 1 H), 7.62 (d, J = 5.0Hz, 1 H).
mp= > 275°C (decomp.)

### Example 29

(2S)-[(4,8-Dioxo-4,8-dihydroxybenzo[1,2-*b*;5,4-*b*']dithiophen-2-carbonyl)-amino]-phenyl-acetic acid methyl ester **XII-1**

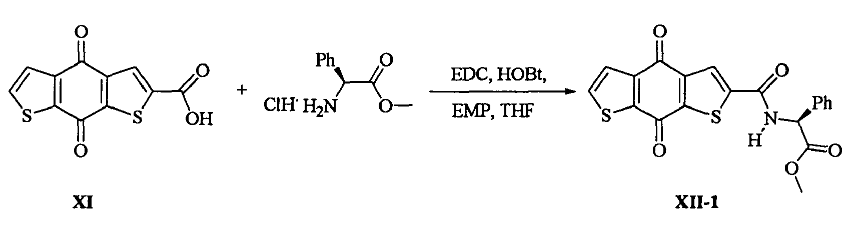

To a stirred solution of **XI** (40.0mg, 0.15mmole) and THF (2ml) were added (S)-phenyl glycine methyl ester HCl salt (31 mg, 0.15mmole), EDC (30.5mg, 0.16mmole), HOBt (21 mg, 0.15mmole) and 4-ethylmorpholine (0.02ml, 0.15mmole). After stirring at R.T for overnight under N₂, the solvent was removed under reduced pressure. CH₂Cl₂ (3ml) was added to the residue and the insoluble solid was removed. The CH₂Cl₂ layer was then washed with saturated NaHCO₃ solution, 1 M KHSO₄, saturated NaHCO₃ solution and H₂O. The organic layer was dried over Na₂SO₄, filtered and concentrated to get crude product. It was purified by column chromatography on silica gel eluting with EA: Hexanes (1:3) to obtain yellow solid **XII-1** (21.1mg , 34 %).
¹H NMR (500MHz, CDCl₃) δ 7.95 (s, 1 H), 7.71 (d, J = 4.3Hz, 1 H), 7.64 (d, J = 4.3Hz, 1H), 7.41-7.30 (m, 5H), 7.16 (d, *J* = 6.5Hz, 1 H), 5.71 (d, *J* = 6.5Hz, 1H), 3.78 (s, 3H).
HRMS Calcd for C₂₀H₁₃NO₅S₂: 411.0235, Found: 411.0229
mp= 211.1~211.9°C

### Example 30

[(4,8-Dioxo-4,8-dihydroxybenzo[1,2-*b*;5,4-*b*']dithiophen-2-carbonyl)-amino]-acetic acid isopropyl ester XII-2

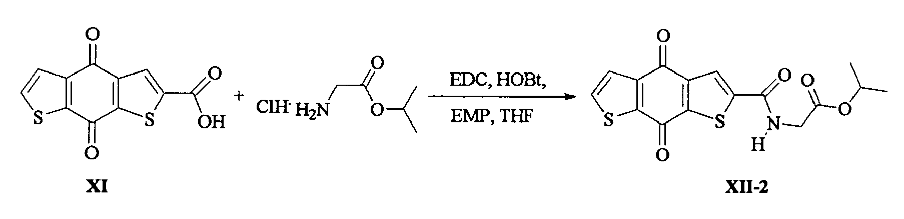

To a stirred solution of **XI** (52.6mg, 0.20mmole) and THF (2ml) were added isopropyl glycinate HCl salt (30.4mg, 0.21 mmole), EDC (40.1 mg, 0.21mmole), HOBt (27.0mg, 0.21mmole) and 4-ethylmorpholine (0.03ml, 0.21 mmole). After stirring at R.T. for overnight under N₂, the solvent was removed under reduced pressure. CH₂Cl₂ (3ml) was added to the residue and the insoluble solid was removed. The CH₂Cl₂ layer was then washed with saturated NaHCO₃ solution, 1 M KHSO₄, saturated NaHCO₃ solution and H₂O. The organic layer was dried over Na₂SO₄, filtered and concentrated to get crude product. It was purified by column chromatography on silica gel eluting with EA: CH₂Cl₂ (1:20) to obtain yellow solid **XII-2** (31.7mg , 44 %).
¹H NMR (500MHz, CDCl₃) δ 7.94 (s, 1 H), 7.71 (d, J = 4.5Hz, 1 H), 7.63 (d, J = 4.5Hz, 1 H), 6.73 (s, 1 H), 5.12 (septet, J = 6.5Hz, 1 H), 4.18 (d, J = 4.5Hz, 2H), 1.29 (d, J = 6.5Hz, 6H).
HRMS Calcd for C₁₆Hₗ₃NO₅S₂: 363.0235, Found: 363.0226
mp= 240.2~240.5°C

### Example 31

(2*S*)-[(4,8-Dioxo-4,8-dihydroxybenzo[1,2-*b*;4,5-*b*']dithiophen-2-carbonyl)-amin o]-propionic acid methyl ester **XII**

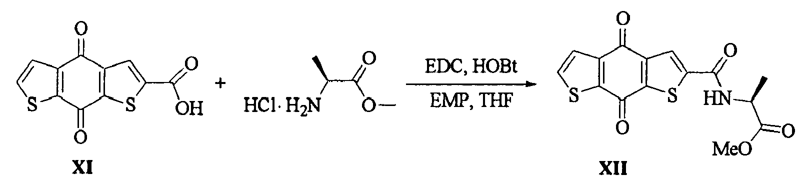

To a stirred solution of **XI** (40.0mg, 0.15mmole) and THF (2ml) were added (S)-alanine methyl ester HCl salt (21 mg, 0.15mmole), EDC (30.5mg, 0.16mmole), HOBt (21 mg, 0.15mmole) and 4-ethylmorpholine (0.02ml, 0.15mmole). After stirring at R.T. for overnight under N₂, the solvent was removed under reduced pressure. CH₂Cl₂ (3ml) was added to the residue and the insoluble solid was removed. The CH₂Cl₂ layer was then washed with saturated NaHCO₃ solution, 1 M KHSO₄, saturated NaHCO₃ solution and H₂O. The organic layer was dried over Na₂SO₄, filtered and concentrated to get crude product. It was purified by column chromatography on silica gel eluting with EA: Hexanes (1:2) to obtain yellow solid XII (27.5mg, 52 %).
¹H NMR (500MHz, CDCl₃) δ 7.92 (s, 1H), 7.72 (d, J = 5.0Hz, 1 H), 7.64 (d, *J* = 5.0Hz, 1 H), 6.75 (d, J = 6.5Hz, 1 H), 4.78-4.72 (m, 1 H), 3.80 (s, 3H), 1.54 (d, J = 7.0Hz, 3H).
HRMS Calcd for C₁₅H₁₁NO₅S₂: 349.0079, Found: 349.0061.
mp= 188.1~188.6°C.

### Example 32

(L)-2-{(4,8-Dioxo-4,8-dihydrobenzo-[1,2-*b*;5,4-*b*']dithiophene-2-carbonyl)-amino)-propionic acid **XIII**

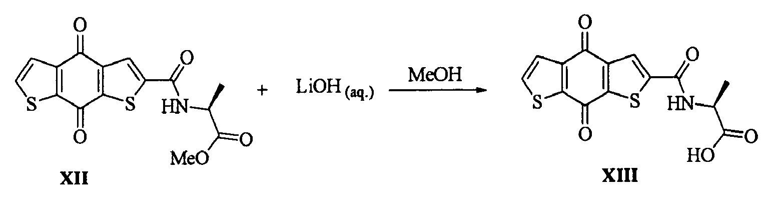

In a 25mL flask were placed compound **XII** (130mg, 0.37mmole) and MeOH (37ml). LiOH (322.5mg) in H₂O (30ml) was then added and reaction mixture stirred at R. T. until hydrolysis completed. HOAc (~1.0ml) was added drop-wise to neutralize. MeOH was removed under vacuum and the residue was treated with aq. Na₂CO₃ and then extracted with EA. The aqueous layer was acidified with dilute HCl solution until pH~4 and the extracted with EA. The EA layer was dried with Na₂SO₄, filtered and concentrated to obtain yellow solid XIII (120mg, 96 %).
¹H NMR (500MHz, CD₃OD) δ 8.25 (s, 1 H), 8.00 (d, *J* = 5.0Hz, 1 H), 7.66 (d, *J* = 5.0Hz, 1 H), 4.58 (q, J = 7.5Hz, 1 H), 1.53 (d, J = 7.5Hz, 3H).
HRMS Calcd for C₁₄H₉NO₅S₂: 334.9922, Found: 334.9931
mp= 239°C (decomp.)

### Example 33

Sodium (L)-2-{(4,8-dioxo-4,8-dihydrobenzo-[1,2-b;5,4-*b*']dithiophene-2-carbonyl)-amino}-propionate **XIII-Na**

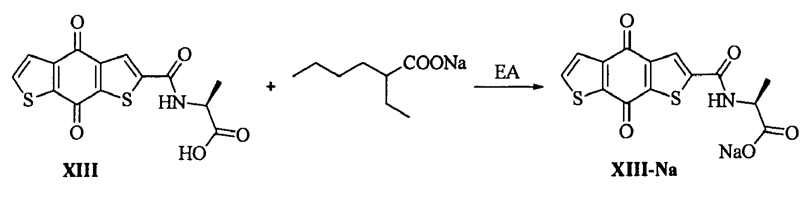

To a stirred solution of **XIII** (50.5mg, 0.15mmole) in EA was added sodium 2-ethylhexanoate (1.2~1.5 eq.) in EA and stirred for overnight. The precipitate was filtered and washed with EA and then dried in vacuum. Yellow solid **XIII-Na** (27.8mg, 52%) was obtained.
¹H NMR (500MHz, D₂O) δ7.93 (br, 1 H), 7.80 (d, J = 5.0Hz, 1 H), 7.44 (d, *J* = 5.0Hz, 1 H), 4.31 (q, J = 6.5Hz, 1 H), 1.50 (d, J = 6.5Hz, 3H).
mp= 200.5°C (decomp.)

### Example 34

Ammonium 4,8-dioxo-4,8-dihydrobenzo-[1,2-*b*;4,5-*b*']dithiophene-2-carboxylate **XIV-NH**_{**4**}

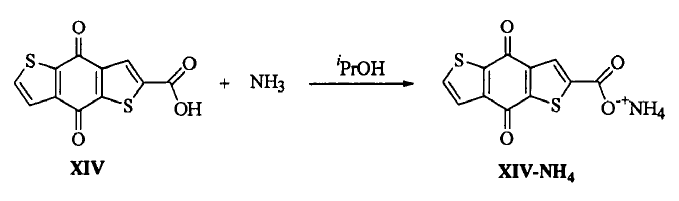

Compound XIV (140mg, 0.53mmole) was added in IPA (20mL) and stirred at 60°C for 30 min. The solid that was not soluble in IPA was filtered. Aqueous ammonium hydroxide (29.5%) was added to the filtrate until basic. After stirring for a while, the solvent was evaporated to yield green-yellow solid **XIV-NH**_{**4**} (68.5mg, 46%)
¹H NMR (500MHz, CD₃OD) δ7.97 (s, 1 H), 7.94 (d, *J* = 5.0Hz, 1 H), 7.64 (d, *J* =5.0Hz, 1 H).
mp> 300 °C

### Example 35

[(4,8-Dioxo-4,8-dihydroxybenzo[1,2-*b*;4,5-*b*']dithiophen-2-carbonyl)-amino]-ac etic acid isopropyl ester **XV**

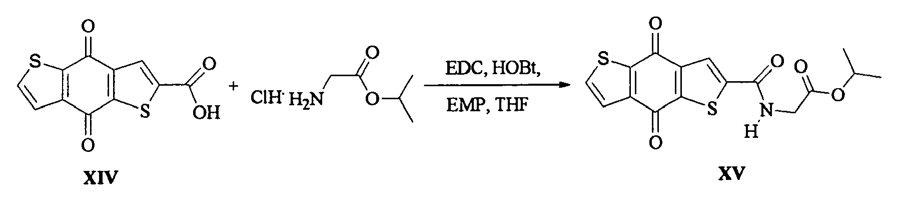

To a stirred solution of **XIV** (244.6mg, 0.90mmole) and THF (8ml) were added isopropyl glycinate HCl salt (140.0mg, 0.92mmole), EDC (190.0mg, 0.99mmole), HOBt (125.0mg, 0.93mmole) and 4-ethylmorpholine (0.12ml, 0.80mmole). After stirring at R.T. for overnight under N₂, the solvent was removed under reduced pressure. CH₂Cl₂ (12ml) was added to the residue and the insoluble solid was removed. The CH₂Cl₂ layer was then washed with saturated NaHCO₃ solution, 1M KHSO₄, saturated NaHCO₃ solution and H₂O. The organic layer was dried over Na₂SO₄, filtered and concentrated to get crude product. It was purified by column chromatography on silica gel eluting with EA: CH₂Cl₂ (1:30) to obtain yellow solid **XV** (75.1 mg , 21 %).
¹H NMR (500MHz, CDCl₃) δ 7.95 (s, 1 H), 7.70 (d, *J* = 5.0Hz, 1 H), 7.65 (d, J = 5.0Hz, 1 H), 6.73 (t, J = 4.5Hz, 1 H), 5.12 (septet, *J* = 6.5Hz, 1 H), 4.18 (d, J = 4.5Hz, 2H), 1.29 (d, J = 6.5Hz, 6H).
HRMS Calcd for C₁₆H₁₃NO₅S₂: 363.0235, Found: 363.0228
mp= 231.8~233.0°C

### Example 36

Methyl (L)-2-{(4,8-Dioxo-4,8-dihydrobenzo-[1,2-*b*;4,5-*b*]dithiophene-2-carbonyl)-amino}-propionate **XV-1**

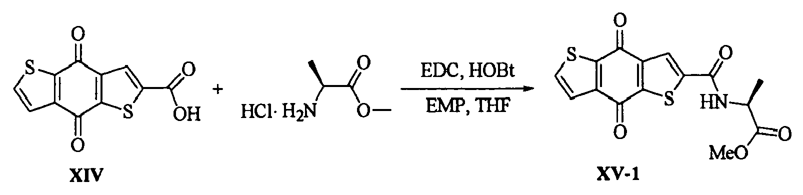

To a stirred solution of **XIV** (79.8mg, 0.30mmole) in THF (10ml) were added (S)-alanine methyl ester HCl salt (42.0mg, 0.30mmole), EDC (61.0mg, 0.32mmole), HOBt (42mg, 0.30mmole) and 4-ethylmorpholine (0.04ml, 0.30mmole). After stirring at R.T. for overnight under N₂, the solvent was removed under reduced pressure. CH₂Cl₂ (3ml) was added to the residue and the insoluble solid was removed. The CH₂Cl₂ layer was then washed with saturated NaHCO₃ solution, 1 M KHSO₄, saturated NaHCO₃ solution and H₂O. The organic layer was dried over Na₂SO₄, filtered and concentrated to get crude product. It was purified by column chromatography on silica gel eluting with EA: Hexanes (1:2) to obtain yellow solid **XV-1** (38.1mg, 36 %).
¹H NMR (500MHz, CDCl₃) δ 7.94 (s, 1 H), 7.71 (d, J = 5.0Hz, 1 H), 7.65 (d, J = 5.0Hz, 1 H), 6.80 (d, J = 6.5Hz, 1 H), 4.75 (qd, J = 7.5, 6.5Hz, 1 H), 3.80 (s, 3H), 1.53 (d, J = 7.5Hz, 3H).
HRMS Calcd for C₁₅H₁₁NO₅S₂: 349.0079, Found: 350.0159 (MH⁺, FAB).
mp= 216.8~217.7 °C

### Example 37

(*L*)-2-{(4,8-Dioxo-4,8-dihydrobenzo-[1,2-*b*;4,5-*b*]dithiophene-2-carbonyl)-amino}-propionic acid **XVI**

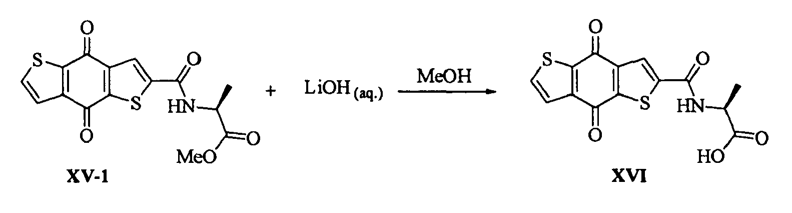

In a 25mL flask were placed compound **XV-1** (17.6mg, 0.05mmole) and MeOH (5ml). LiOH (45.0mg) in H₂O (4ml) was then added and reaction mixture stirred at R.T. until hydrolysis completed. HOAc (~0.14ml) was added drop-wise to neutralize. MeOH was removed under vacuum and the residue was treated with aq. Na₂CO₃ and then extracted with EA. The aqueous layer was acidified with dilute HCl solution until pH~4 and the extracted with EA. The EA layer was dried with Na₂SO₄, filtered and concentrated to obtain yellow solid XVI (16.8mg, 99 %).
¹H NMR (500MHz, CDCl₃) δ 7.96 (s, 1 H), 7.62 (d, J = 5.0Hz, 1 H), 7.47 (d, J = 5.0Hz, 1 H), 4.47 (q, J = 7.5Hz, 1 H), 1.36 (d, J = 7.5Hz, 3H).
mp= 227.4~228.3 °C

### Example 38

Sodium (*L*)-2-{(4,8-dioxo-4,8-dihydrobenzo-[1,2-*b*;4,5-*b*]1dithiophene-2-carbonyl)-amino}-propionate **XVI-Na**

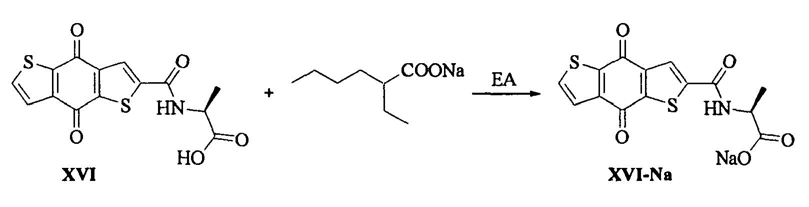

To a stirred solution of **XVI** (73.8mg, 0.22mmole) in EA was added sodium 2-ethylhexanoate (1.2~1.5 eq.) in EA and stirred for overnight. The precipitate was filtered and washed with EA and then dried in vacuum.
Yellow-green solid **XVI-Na** (47.5mg, 60%) was obtained.
¹H NMR (500MHz, CD₃OD δ8.25 (s, 1 H), 7.96 (d, J = 5.0Hz, 1 H), 7.65 (d, J = 5.0Hz, 1 H), 4.43 (q, J = 7.0Hz, 1 H), 1.48 (d, J = 7.0Hz, 3H).
mp> 275 °C.

### Example 39

4,8-dioxo-4,8-dihydrobenzo [1,2-*b*;4,5-*b*']dithiophene-2-carboxylate-(2-dimethylamino-ethyl)-amide **XV-2**

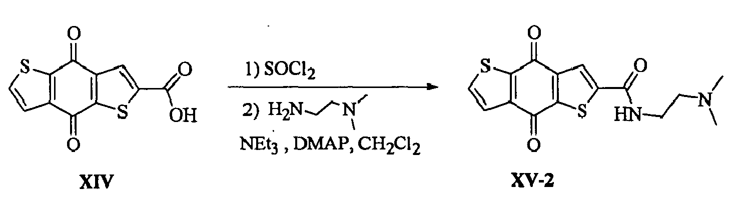

Compound **XIV** (0.6086g, 2.0mmole), SOCl₂ (50ml) along with a small amount of NaCl was heated to reflux for 2 hrs. The excess SOCl₂ was removed and CH₂ Cl₂was added to the residue. NaCl was filtered and the filtrate was concentrated to yield crude acid chloride (0.5453g). The acid chloride obtained was used directly without purification. It was diluted with CH₂Cl₂ (40ml) and added to the *N,N*-dimethylethylenediamine (16ml), Et₃N (20ml) and CH₂Cl₂ (40ml) mixture and stirred at R.T. for 12 hrs. Water was added and the reaction mixture extracted with CH₂Cl₂. The CH₂Cl₂ layer was concentrated and crude product was purified through column using MeOH: CH₂Cl₂ =1:10 as the eluent. Yellow solid **XV-2** (255.2mg, 33 %) was obtained. ¹H NMR (500MHz, CD₃OD) δ 8.13 (s, 1 H), 7.97 (d, J = 5.0Hz, 1H), 7.65 (d, J = 5.0Hz, 1 H), 3.55 (t, J = 6.5Hz, 2H), 2.65 (t, J = 6.5Hz, 2H), 2.37 (s, 6H). HRMS Calcd for C₁₅H₁₄N₂O₃S₂: 334.0446, Found: 334.0641.
mp= 235.5~236.2°C

### Example 40

4,8-dioxo-4,8-dihydrobenzo [1,2-*b*;4,5-*b*']dithiophene-2-carboxylate-(2-dimethyl-aminoethyl)-amide phosphoric acid salt **XV-2-H**_{**3**}**PO**_{**4**}

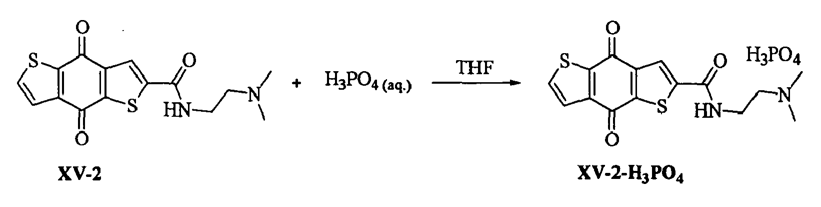

To a stirred solution of **XV-2** (100mg, 0.30mmole) in THF was added 85% H₃PO₄ (1.2~1.5 eq.) and stirred for overnight. The precipitate was filtered and washed with THF and then dried in vacuum. Yellow solid **XV-2-H**_{**3**}**PO**_{**4**} (120mg, 93%) was obtained.
¹H NMR (500MHz, D₂O) δ8.02 (br, 1 H), 7.82 (br, 1 H), 7.54 (br, 1 H), 3.88 (br, 2H), 3.52 (br, 2H), 3.08 (br, 6H).
mp= 233°C (decomp.)

**Cytotoxicity Assays Method A (HUVEC, MCF-7, HT-29, Hep 3B and NCI-H460)** [S. A. Ahmed, R. M. Gogal Jr., and J. E. Walsh, Journal of Immunological Methods 170: 211-224, (1994); M. R. Boyd, Status of the NCI preclinical antitumor drug discovery screen. (Published by J. B. Lippincott Company, Philadelphia, PA 19105, USA) Principles & Practices of Oncology Updates 3 # 10: 1-12, (1989); M. R. Boyd, et al. Data display and analysis strategies for the NCl disease-oriented *in vitro* antitumor drug screen. In: Cytotoxic anti-cancer drugs: models and concepts for drug discovery and development. Boston: KluwerAcademic, Pages: 11-34, (1992)]

### 1. Materials and Equipment

### (1) Test substance and Dosing pattern

The test compounds were dissolved in 100% DMSO and then diluted with sterile distilled water to obtain initial working solutions of 20000, 2000, 200, 20 and 2 µM in 80% DMSO. A 200 fold dilution was further made in culture media to generate final assay concentrations of 100, 10, 1, 0.1 and 0.01 µM in 0.4% DMSO.

### (2) Cell Culture Media

| Cell Lines | Culture Medium |
|---|---|
| HUVEC | Endothelial Cell Growth Medium, 90%; Fetal Bovine Serum, 10% |
| MCF-7 | Minimum Essential Medium, 90%; Fetal Bovine Serum, 10% |
| HT-29 | McCoy's 5A medium, 90%; Fetal Bovine Serum, 10% |
| Hep 3B | Minimum Essential Medium, 90%; Fetal Bovine Serum, 10% |
| NCl-H460 | RPMI 1640, 90%; Fetal Bovine Serum, 10% |

| | |
|---|---|
| All of media were supplemented with 1% Antibiotic-Antimycotic. | |

### (3) Cell Lines

| Cell Name | Source | Type of Cell Line |
|---|---|---|
| HUVEC | ATCC CRL-1730 | Human umbilical vein endothelial cells |
| MCF-7 | ATCC HTB-22 | Breast adenocarcinoma, pleural effusion, human |
| HT-29 | ATCC HTB-38 | Adenocarcinoma, colon, moderately |
| | | well-differentiated grade II, human |
| Hep 3B | ATCC HB-8064 | Hepatocellular carcinoma, liver, human |
| NCI-H460 | ATCC HTB-177 | Large cell carcinoma, lung, human |

| | | |
|---|---|---|
| All of the human tumor cell lines and HUVEC were obtained from American Type Culture Collection (ATCC). The cells were all incubated at 37°C with 5 % CO₂ in air atmosphere. | | |

### (4) Chemicals

AlamarBlue (Biosource, USA), Antibiotics-Antimycotic (GIBCO BRL, USA), Dimethylsulfoxide (Merck, Germany), Endothelial Cell Growth Medium (CELL APPLICATIONS, INC., USA), Fetal Bovine Serum (HyClone, USA), McCoy's 5A Medium (GIBCO BRL, USA), Minimum Essential medium (GIBCO BRL, USA), Mitomycin (Kyowa, Japan) and RPMI 1640 (HyClone, USA).

### (5) Equipment

CO₂ Incubator (Forma Scientific Inc., USA), Centrifuge 5810R (Eppendorf, Germany), Hemacytometer (Hausser Scientific Horsham, USA), Inverted Microscope CK-40 (Olympus, Japan), System Microscope E-400 (Nikon, Japan), Spectrafluor Plus (Tecan, Austria) and Vertical Laminar Flow (Tsao Hsin, R. O. C.).

### 2. Methods

### (1) Evaluation of anti-proliferative activity for test substances

Aliquots of 100 µl of cell suspension (about 1.5 - 3.0 x 10³/well) were placed in 96-well microtiter plates in an atmosphere of 5% CO₂ at 37°C. After 24 hours, 100 µl of growth medium and 1 µl of test solution or vehicle (80% DMSO) were added respectively per well in duplicate for an additional 72-hour incubation. Thus, the final concentration of DMSO was 0.4%. The test compounds, IIa-1, IIb-1 and VII-1 were evaluated at concentrations of 100, 10, 1, 0.1 and 0.01 µM. At the end of incubation, 20 µl of alamarBlue 90% reagent was added to each well for another 6-hour incubation before detection of cell viability by fluorescent intensity. Fluorescent intensity was measured using a Spectraflour Plus plate reader with excitation at 530 nm and emission at 590 nm.

### (2) Determination of IC₅₀, TGI and LC₅₀

The measured results was calculated by the following formula: $PG ( % ) = 100 \times ( {Mean F}_{test} - {Mean F}_{time 0} ) / ( {Mean F}_{ctrl} - {Mean F}_{time 0} ) ]$
If (Mean Fₜₑₛₜ - Mean Fₜᵢₘₑ₀) < 0, then$PG ( % ) = 100 \times ( {Mean F}_{test} - {Mean F}_{time 0} ) / ( {Mean F}_{time 0} - {Mean F}_{blank} )$
wherein PG represents percent growth;
Mean Fₜᵢₘₑ₀ = The average of 2 measured fluorescent intensities of reduced alamarBlue at the time just before exposure of cells to the test substance;
Mean Fₜₑₛₜ = The average of 2 measured fluorescent intensities of alamarBlue after 72-hour exposure of cells to the test substance;
Mean F_{ctrl} = The average of 2 measured fluorescent intensities of alamarBlue after 72- hour incubation without the test substance;
Mean F_{blank} = The average of 2 measured fluorescent intensities of alamarBlue in medium without cells after 72-hour incubation.

A decrease of 50% or more (≥50%) in fluorescent intensity relative to the vehicle-treated control indicates significant cytostatic or cytotoxic activity, and semi-quantitative values for IC₅₀, TGI and LC₅₀ were then determined by nonlinear regression using GraphPad Prism (GraphPad Software, USA).

IC₅₀ (50% Inhibition Concentration): Test compound concentration where the increase from time₀ in the number or mass of treated cells was only 50% as much as the corresponding increase in the vehicle-control at the end of experiment.

TGI (Total Growth Inhibition): Test compound concentration where the number or mass of treated cells at the end of experiment was equal to that at time₀.

LC₅₀ (50% Lethal Concentration): Test compound concentration where the number or mass of treated cells at the end of experiment was half that at time.

Cytotoxicity Assays Method B (HL-60) [H. M. Chen et al. *Chin. Pharm. J.* 53, 157-167 (2001)]

### Cells and Culture

Human promyeloid leukemia HL-60 cells were obtained from the American Type

Culture Collection (Manassas, VA, USA) and the Culture Collection and Research Center(CCRC) (Tai wan, ROC). Human promyeloid leukemia HL-60 cells were cultured in suspension in RPMI-1640 medium (GIBCO, Grand Is land, USA), containing 10 % fetal bovine se rum (GIBCO; Grand Is land, USA), 100 unit/mL penicillin, 100 mUmL streptomycin and 1 % L-glutamine at 37 °C in a humidified at mo sphere of 5 % CO2 in air. Cells were split every day to maintain the cell numbers between 2-5 x 10⁵/mL. Cell numbers were assessed by the standard procedure of leukocyte counting using a hemocytometer and cell viability was checked by the ability of cells to exclude Trypan blue.

### MTT Proliferation Assay

Cellular proliferation was determined by the MTT [3-(4,5-dimethylthiazol- 2- yl)-2,5-diphenyltetrazolium bromide] as say. Briefly, 10 mL of MTT (5 mg/mL) was added to each well of 96-well plates containing 1 x 10⁵ cells after treatment with different concentrations of samples for 4 days. The reaction was stopped after 2 h by adding 100 mL of 0.04 N HCl in isopropanol and the OD570 nm was determined by a minicolorimetric reader. Each concentration treatment was performed in triplicate.

**Cytotoxicity Assays Method C (KB, KB-VIN, AS49, HCT-8, PC-3) [K. H.** Lee et. al. Planta Med. 54, 308-312 (1998), Monk A et. al. J. Natl. Cancer Inst. 83, 757-766 (1991).] The cell lines included epidermoid caninoma of the nasopharynx (KB), p-gp-expressing epidermoid caninoma of the nasopharynx (KB-VIN), lung carcinoma (A-549), ileocecal carinoma (HCT-8) and prostate cancer (PC-3) cell lines. The cytotoxic effects of each compounds were obtained as IC₅₀ in microgram per ml, the concentration that give 50% inhibition of cell growth after 72hrs of continuous exposure-variation was less than 5% between replicates.

### Anticancer activity

The assays were used to detect changes in cell proliferation based on the ability of viable cells to cause alamarBlue to change from its oxidized to a reduced form. With the results obtained from the alamarBlue reaction, cell proliferation can be quantified and metabolic activity of viable cell can be examined.

The succinate derivatives of compounds **IIa-1**, **IIb-1** and **VII-1** were tested for possible effect on the proliferation of four human tumor cell lines ~MCF-7 (breast), HT-29 (colon), Hep3B (liver), and NCl-H 460 (lung) as well as one human umbilical vein endothelial cell (HUVEC) at assay concentration from 0.01 to 100 µM, through serial 10-fold dilution.

The IC₅₀(50% inhibition concentration), TGI (total growth inhibition) and LC₅₀ (50% lethal concentration) of the three tested compounds were summary in **Table 1.**

As shown in **Table 1**, the three tested compounds showed potent cytotoxicity in vitro against NCI-H 460 cell line. Compounds **IIa-1** and **IIb-1** also had significant activity against MCF-7 and Hep3B cell lines. These two compounds displayed moderate and little activity against HT-29 cell line. Compound VII-1 showed significant cytotoxicity against MCF-7 and Hep3B and moderate activity against HT-29 cell line. On the other hand, all the three tested compounds were essentially inactive or showed little activity in HUVEC assay.

Compound **IIa-1, IIa-2, IIb-1, IIb-2, VII-1, VII-2, IX** and **XV-2-H**_{**3**}**PO**_{**4**} were evaluated for their cytotoxicity in vitro against human promyelocytic leukemia (HL-60) cell line. As shown in Table 2. All of these tested compounds showed significant cytotoxicity. Among them, **XV-2-H**_{**3**}**PO**_{**4**} was the most potent one. There fore this compound was selected for further evaluation.

Mono-[1-(4,8-dioxo-4,8-dihydrobenzo[1,2-b:5,4-b'] dithiophen-2-yl)-methyl]succinate **(IIa-1),** mono-[1 -(4,8-dioxo-4,8-dihydrobenzo[1,2-b:5,4-b'] dithiophen-2-yl)-methyl] pentanedioate (IIa-2), mono-[1-(4,8-dioxo-4,8-dihydrobenzo[1,2-b:5,4-b'] dithiophen-2-yl)-ethyl]succinate (IIb-1), mono-[1 -(4,8-dioxo-4,8-dihydrobenzo[1,2-b:5,4-b'] dithiophen-2-yl)-ethyl] pentanedioate (IIb-2), ethyl 4,8-dioxo-4,8-dihydroxybenzo[1,2-b:5,4-b']-dithiophen-2-yl-methoxy acetate **(IVa),** mono-[1-(4,8-dioxo-4,8-dihydroxybenzo[1,2-b;4,5-b']dithiophen-2-yl)-ethyl] succinate **(VII-1),** mono-[1-(4,8-dioxo-4,8-dihydroxybenzo[1,2-b;4,5-b']dithiophen-2-yl)-ethyl] pentanedioate (VII-2), 1-(4,8-Dioxo-4,8-dihydroxybenzo[1,2-b:4,5-b']-dithiophen-2-yl-ethoxy)-acetate (IX), 4,8-dioxo-4,8-dihydrobenzo-[1,2-b;5,4-b']dithiophene-2-carboxylic acid **(XI)**, 4,8-dioxo-4,8-dihydrobenzo-[1,2-*b*;4,5-*b*']dithiophene-2-carboxylic acid **(XIV),** 4,8-dioxo-4,8-dihydrobenzo-[1,2-b;4,5-b']dithiophene-2-carboxylate-(2-dimethylamino-ethyl)-amide phosphate **(XV-2-H**_{**3**}**PO**_{**4**}**),** (L)-2-{(4,8-Dioxo-4,8-dihydrobenzo-[1,2-b;4,5-b']dithiophene-2-carbonyl)-ami no}-propionic acid (**XVI**), and their salts were evaluated for their cytotoxicity in vitro against epidermal carcinoma of the nasopharynx (KB), p-gp-expressing epidermoid carcinoma of the nasopharynx (KB-VIN), lung carcinoma (A-549), ileocecal carcinoma (HCT-8) and prostate cancer (PC-3). As shown in Table 2, Most of these tested compounds showed significant cytotxicity. Among them, **XV-2-H**_{**3**}**PO**_{**4**} was the most promising agent. In addition, compounds IIb-1, **IIb-2, VII-1** and **VII-2** displayed potent activity against the MDR cell line (KB-VIN) and the androgen-insensitive prostate cancer cell line (PC-3). Therefore, compounds **XV-2-H**_{**3**}**PO**_{**4**}**, IIb-1, IIb-2 VII-1**and **VII-2** were selected for further pharmacological studies. In summary, the present invention demonstrated remarkable therapeutical potential for benzodithiophenone analogs in the treatment of cancers.

The present invention also discloses a hydrophilic compound having the following structures (F) or (G), or a pharmaceutically acceptable salt thereof: 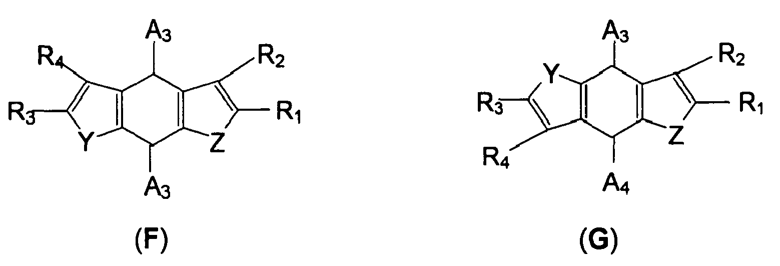 wherein A₃ and A₄ independently are -U-C(O)-(CH₂)ₙCOOH, -U-(CH₂)ₙ-COOH, -U(CH₂)n-NR₅R₆, 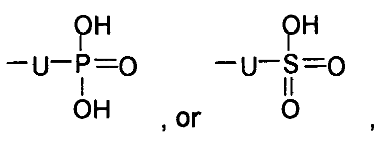 wherein U is O, S, or NH, R₅ is H or alkyl; R₆ is H or alkyl; and n is 1-5; and Y, Z, R₁, R₂, R₃ and R₄ are defined as above.

The present invention also discloses a method for treating a tumor, said method comprising administering to a subject in need of treatment a compound having the formulas (**F**) or (**G**) as defined above, or a pharmaceutically acceptable salt thereof, in an amount effective to treat said tumor.

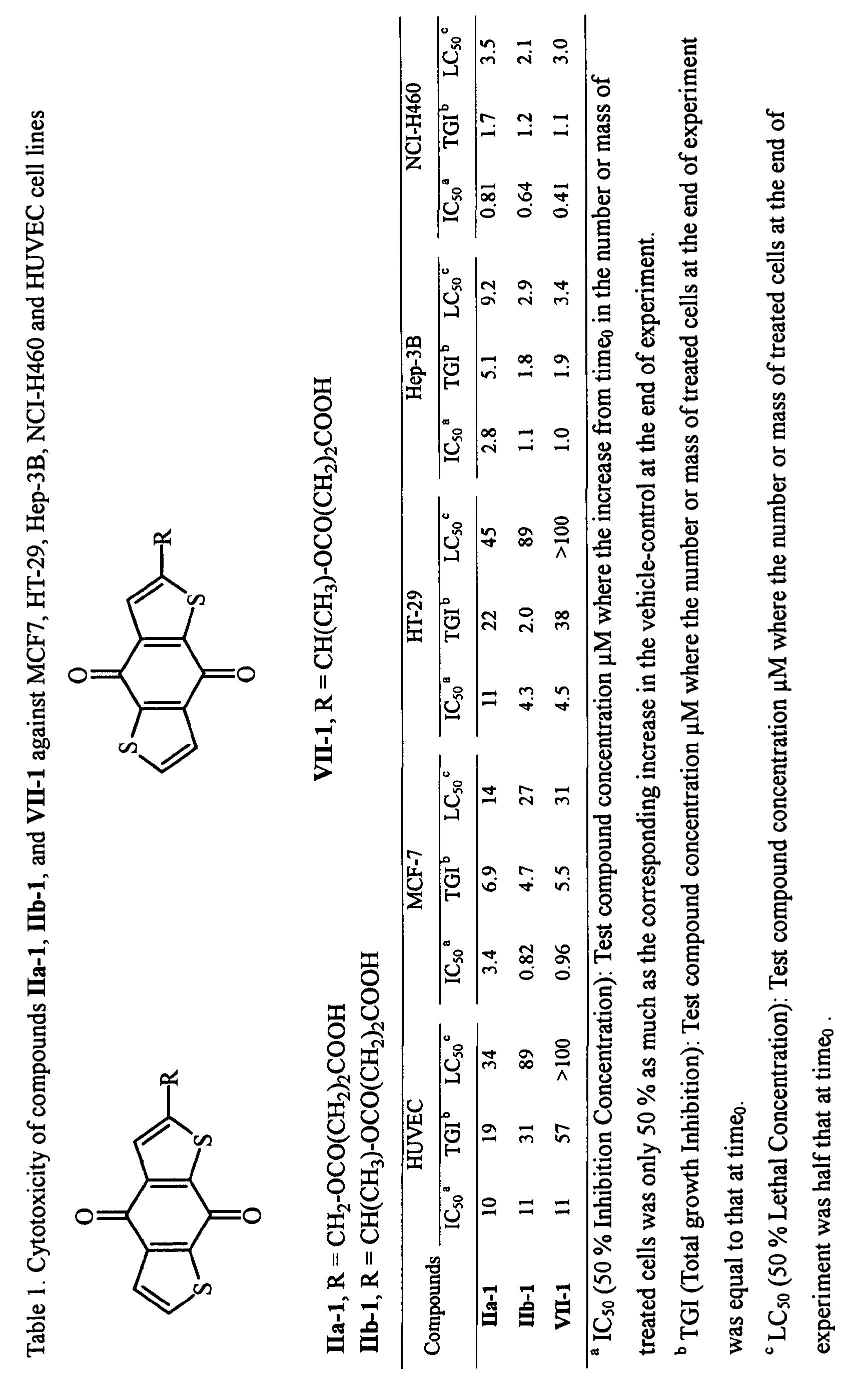

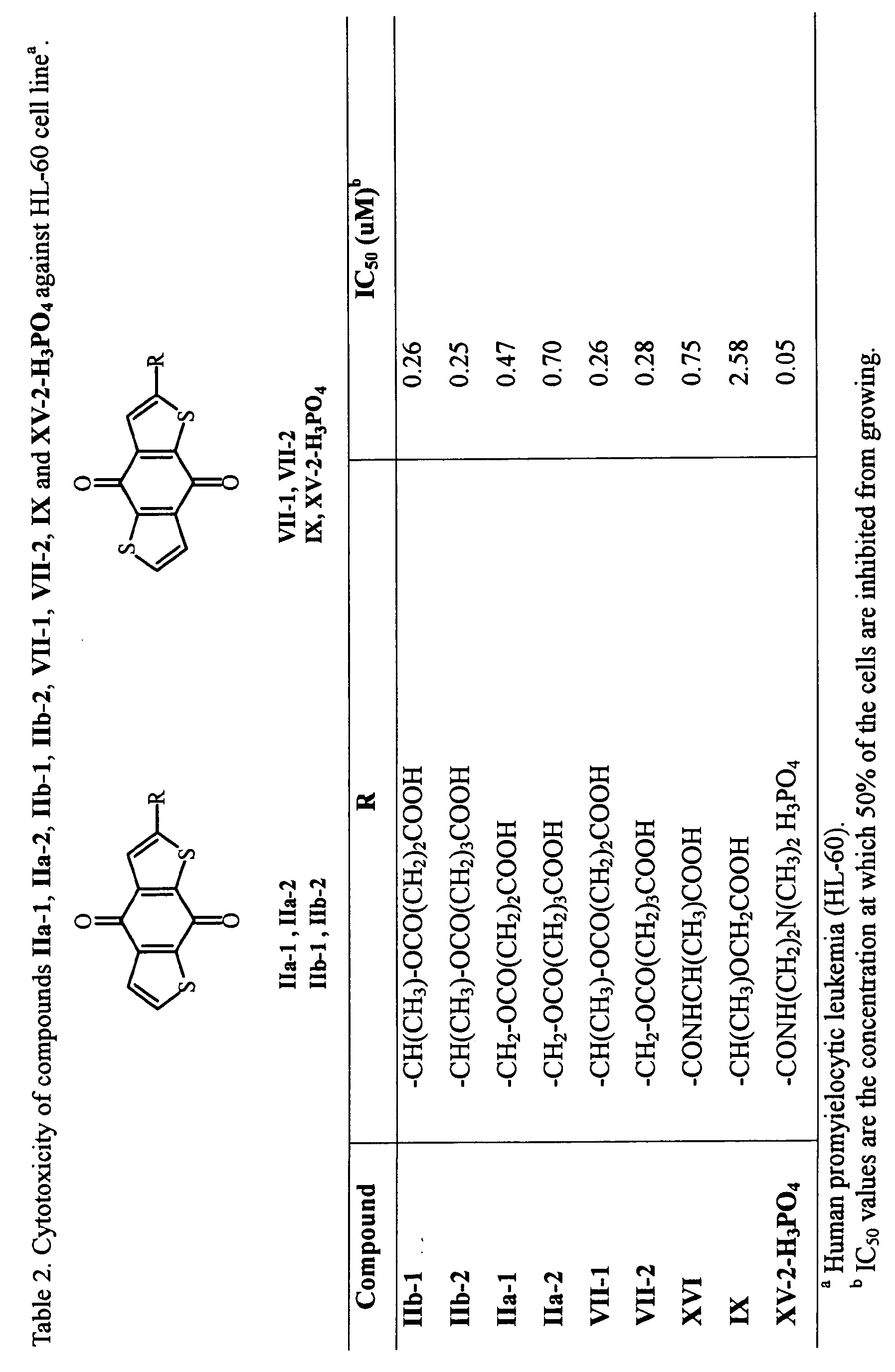

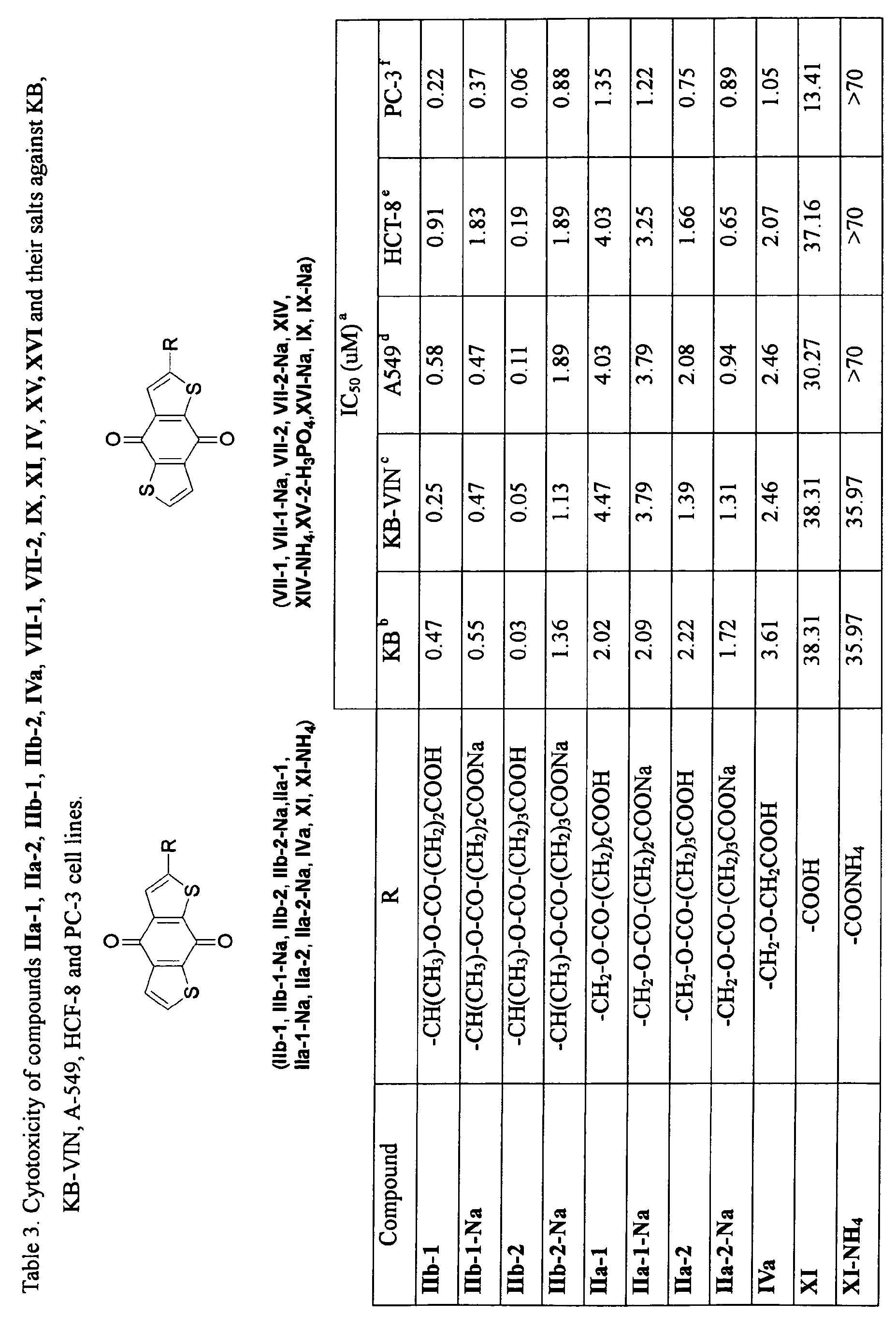

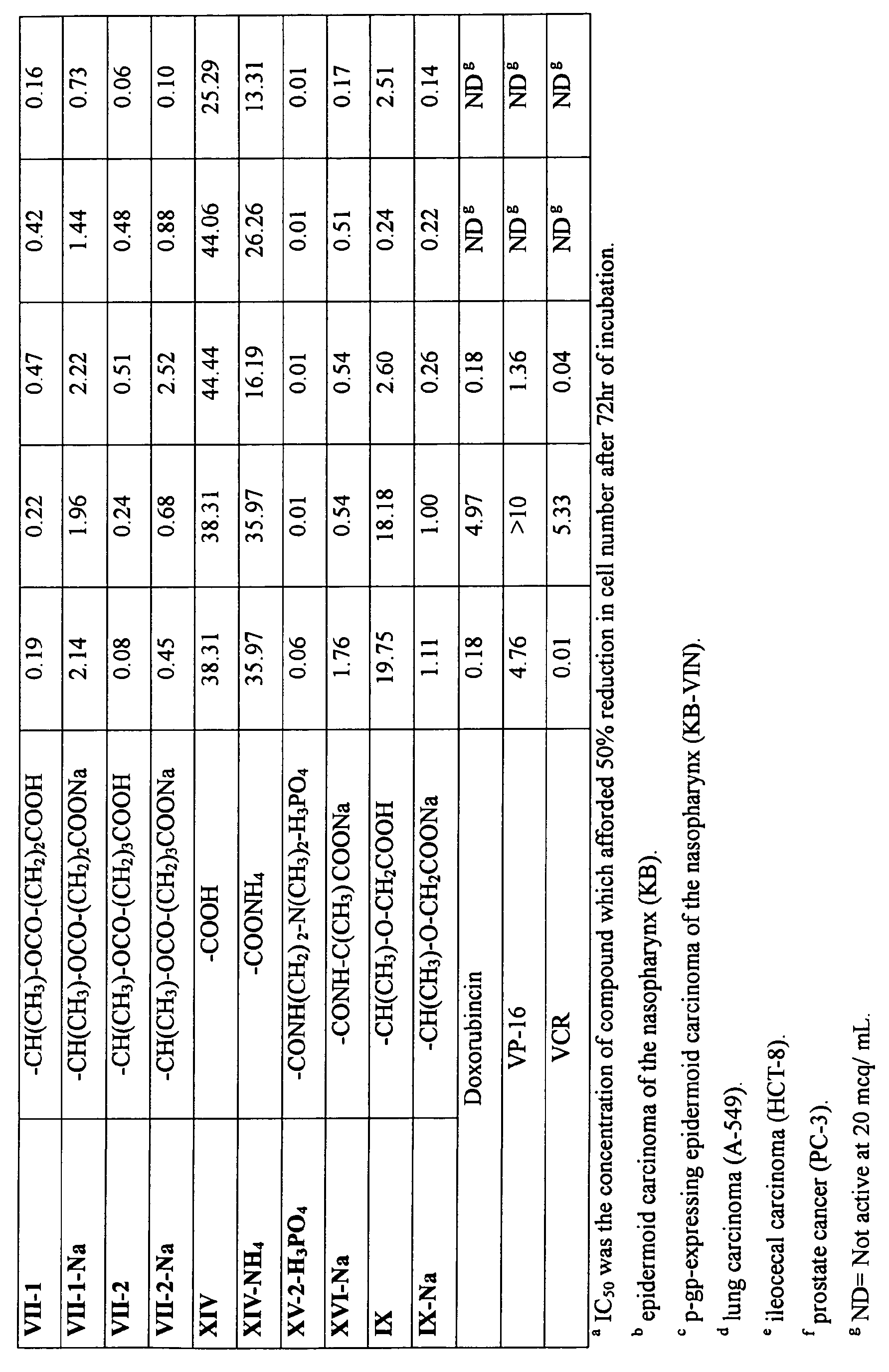

The water solubility of some of the synthesized compounds were tested and summarized as below.

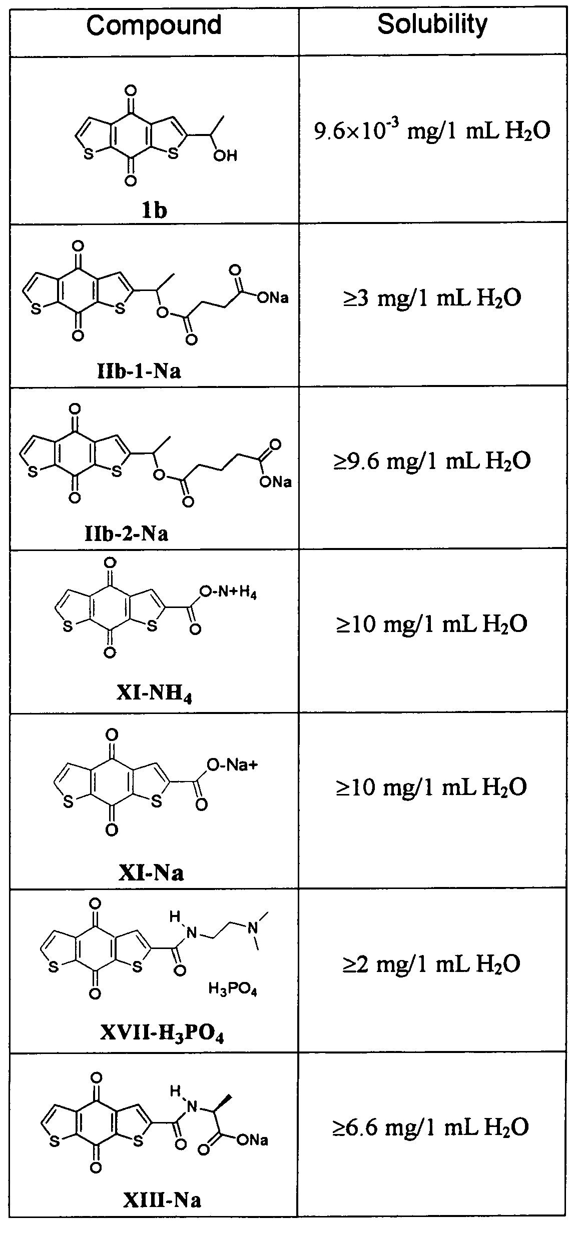

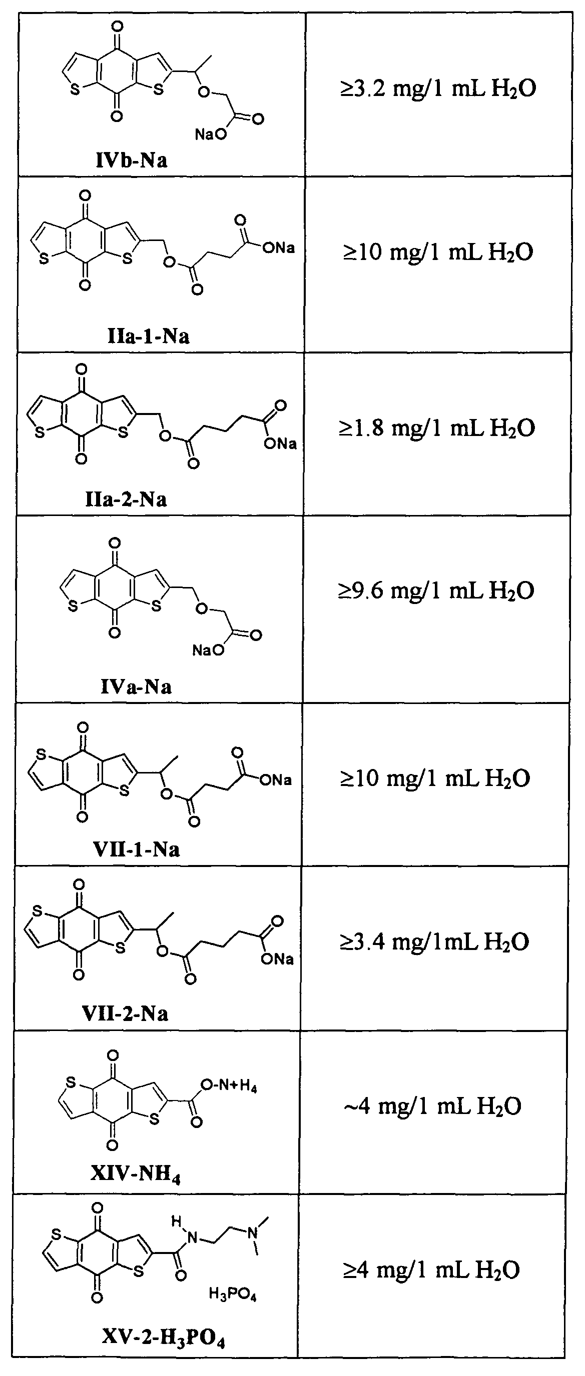

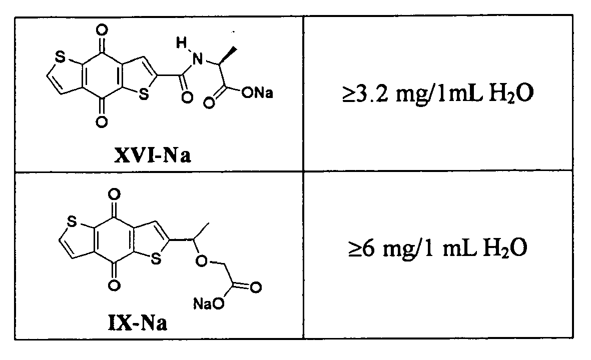

## Claims

1. A derivative of 4,8-dihydrobenzodithiophene-4,8-dione having the formulas (D) or (E): 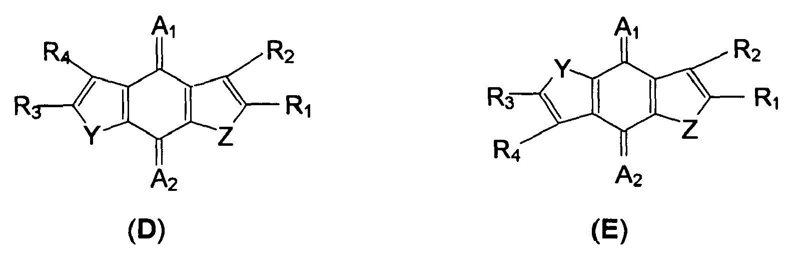 wherein Y and Z independently are O, S, -NH-, or Se;
A₁ and A₂ independently are O, S, or NR₅, wherein R₅ is H or alkyl;
R_{1'} R₂, R₃ and R₄ independently are H, alkyl,
-CR₅R₆-X-C(O)-(CH₂)ₙCOOH, -CR₅R₆-X-(CH₂)ₙCOOH,
-CR₅R₆-X-(CH₂)ₙNR₅R₆, -C(O)-NR₆-(CR₅R₆)ₙCOOH,
-C(O)-NR₆-(CR₅R₆)ₙNR₅R₆, -CR₅R₆-C(O)-(CH₂)ₙCOOH,
-CR₅R₆-X-C(O)-(CH₂)ₙNR₅R₆, -C(O)-(CR₅R₆)ₙCOOH, -C(O)-(CR₅R₆)ₙNR₅R₆,
-CR₅=N-(CH₂)ₙCOOH, -CR₅=N-(CH₂)ₙNR₅R₆, -CR₅=NOH, or 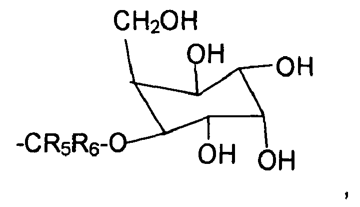 wherein X is O, S, or NH; R₅ is defined as above; R₆ is H or alkyl; and n is 1-5; subject to the proviso that at least one of R_{1'} R₂, R₃ and R₄ is a radical other than hydrogen and alkyl; or a pharmaceutically acceptable salt thereof.

2. The derivative as defined in claim 1, wherein R₂, R₃ and R₄ are H, and R₁ is -CR₅R₆-X-C(O)-(CH₂)ₙCOOH, -CR₅R₆-X-(CH₂)ₙCOOH,
-CR₅R₆-X-(CH₂)ₙNR₅R₆, -C(O)-NR₆-(CR₅R₆)ₙCOOH, or
-C(O)-NR₆-(CR₅R₆)ₙNR₅R₆, wherein X, R₅, R₆ and n are defined as in claim 1.

3. The derivative as defined in claims 1 or 2, wherein Y and Z are S.

4. The derivative as defined in claims 1, 2 or 3, wherein A₁ and A₂ are O.

5. The derivative as defined in claim 4, wherein X is O.

6. The derivative as defined in claim 5, wherein R₅ and R₆ independently are H or methyl.

7. The derivative as defined in claim 6, wherein R₁ is
-CR₅R₆-X-C(O)-(CH₂)ₙCOOH.

8. The derivative as defined in claim 6, wherein R₁ is
-CR₅R₆-X-(CH₂)ₙCOOH.

9. The derivative as defined in claim 6, wherein R₁ is
-CR₅R₆-X-(CH₂)ₙNR₅R₆.

10. The derivative as defined in claim 6, wherein R₁ is
-C(O)-NR₆-(CR₅R₆)ₙCOOH.

11. The derivative as defined in claim 6, wherein R₁ is
-C(O)-NR₆-(CR₅R₆)ₙNR₅R₆.

12. The derivative as defined in claim 6 having the formula (D).

13. The derivative as defined in claim 6 having the formula (E).

14. The derivative as defined in claim 6, which is mono-[1-(4,8-dioxo-4,8-dihydrobenzo[1,2-*b*:5,4-*b*] dithiophen-2-yl)-methyl]succinate (IIa-1), mono-[1-(4,8-dioxo-4,8-dihydrobenzo[1,2-*b*:5,4-*b*] dithiophen-2-yl)-ethyl]succinate (IIb-1), or mono-[1-(4,8-dioxo-4,8-dihydrobenzo[1,2-*b*:5,4-*b*']dithiophen-2-yl)-ethyl] pentanedioate (IIb-2); mono-[1-(4,8-dioxo-4,8-dihydroxybenzo[1,2-*b*;4,5-*b*']dithiophen-2-yl)-ethyl] succinate (VII-1), mono-[1-(4,8-dioxo-4,8-dihydroxybenzo[1,2-*b*;4,5-*b*']dithiophen-2-yl)-ethyl] pentanedioate (VII-2), or 4,8-dioxo-4,8-dihydrobenzo[1,2-*b*;4,5-*b*]dithiophene-2-carboxylate-(2-dimethylamino-ethyl)-amide (XV-2).

15. Use of a derivative of 4,8-dihydrobenzodithiophene-4,8-dione having the formulas (D) or (E) as defined in any one of claims 1 to 14, or a pharmaceutically acceptable salt thereof, in the preparation of a medicament for treating a tumor.

16. The use as defined in claim 15, wherein said tumor is selected from the group consisting of non-small cell lung cancer, breast cancer, nasopharynx carcinoma, prostate cancer, colon cancer, hepatoma, ileocecal carcinoma, leukemia and central nervous system cancers.

17. The use as defined in claim 16, wherein said tumor is selected from the group consisting of non-small cell lung cancer, breast cancer, nasopharynx carcinoma and prostate cancer.
